# EUROPEAN PATENT APPLICATION

(11) **EP 1 655 371 A1**
(43) Date of publication of application: **10.05.2006**
(21) Application number: 04742091.4
(22) Date of filing: 19.07.2004
(51) Int. Cl.: C12N 15/67, C07K 16/18

(54) **SYSTEM FOR THE PRODUCTION OF DIMERIC PROTEINS BASED ON THE TRANSPORT SYSTEM OF HEMOLYSIN OF ESCHERICHIA COLI**

(30) Priority: 31.07.2003 ES 200301830
(71) Applicant: CONSEJO SUPERIOR DE INVESTIGACIONES CIENTIFICAS, 28006 Madrid (ES)
(72) Inventor: LORENZO PRIETO, Victor de Centro Nac. Biotech., 28049 Madrid (ES); FERNANDEZ HERRERO, Luis A. Centro Nac. Biotech., 28049 Madrid (ES)
(74) Representative: ABG Patentes, S.L.
(86) International application number: PCT/ES2004/070053
(87) International publication number: WO 2005/012532

(57) **Abstract**

The system comprises a DNA construct comprising: a) a first nucleic acid sequence containing the nucleotide sequence coding for a product of interest; b) a second nucleic acid sequence containing the nucleotide sequence coding for a dimerization domain; and c) a third nucleic acid sequence containing the nucleotide sequence coding for *Escherichia coli* α-hemolysin (HlyA) or for a fragment of said protein comprising the recognition signal of the *E. coli* hemolysin (Hly) transport system secretion mechanism. It is applicable in producing recombinant dimeric proteins.

SEQUENCE LISTING

<110> Consejo Superior de Investigaciones Científicas

<120> System for producing dimeric proteins based on the Escherichia coli hemolysin transport system

<160> 8

<170> PatentIn version 2.0

<210> 1
<211> 654
<212> DNA
<213> Escherichia coli
<400> 1
ggaaaattct cttgctaaaa atgtattatc cggtggaaaa ggtaatgaca agttgtacgg 60
cagtgaggga gcagacctgc ttgatggcgg agaagggaat gatcttctga aaggtggata 120
tggtaatgat atttatcgtt atctttcagg atatggccat catattattg acgatgaagg 180
ggggaaagac gataaactca gtttagctga tatagatttc cgggacgttg cctttaagcg 240
agaagggaat gacctcatta tgtataaagc tgaaggtaat gttctttcta ttggccacaa 300
aaatggtatt acatttaaaa actggtttga aaaagagtca gatgatctct ctaatcatca 360
gatagagcag atttttgata aagacggcag ggtaatcaca ccagattctc ttaaaaaagc 420
atttgaatat cagcagagta ataacaaggt aagttatgtg tatggacatg atgcatcaac 480
ttatgggagc caggacaatc ttaatccatt aattaatgaa atcagcaaaa tcatttcagc 540
tgcaggtaac ttcgatgtta aggaggaaag atctgccgct tctttattgc agttgtccgg 600
taatgccagt gatttttcat atggacggaa ctcaataact ttgacagcat cagc 654

<210> 2
<211> 218
<212> PRT
<213> Escherichia coli
<400> 2
Glu Asn Ser Leu Ala Lys Asn Val Leu Ser Gly Gly Lys Gly Asn Asp
1               5                   10                  15

Lys Leu Tyr Gly Ser Glu Gly Ala Asp Leu Leu Asp Gly Gly Glu Gly
            20                  25                  30

Asn Asp Leu Leu Lys Gly Gly Tyr Gly Asn Asp Ile Tyr Arg Tyr Leu
        35                  40                  45

Ser Gly Tyr Gly His His Ile Ile Asp Asp Glu Gly Gly Lys Asp Asp
    50                  55                  60

Lys Leu Ser Leu Ala Asp Ile Asp Phe Arg Asp Val Ala Phe Lys Arg
65                  70                  75                  80

Glu Gly Asn Asp Leu Ile Met Tyr Lys Ala Glu Gly Asn Val Leu Ser
                85                  90                  95

Ile Gly His Lys Asn Gly Ile Thr Phe Lys Asn Trp Phe Glu Lys Glu
            100                 105                 110

Ser Asp Asp Leu Ser Asn His Gln Ile Glu Gln Ile Phe Asp Lys Asp
        115                 120                 125

Gly Arg Val Ile Thr Pro Asp Ser Leu Lys Lys Ala Phe Glu Tyr Gln
    130                 135                 140

Gln Ser Asn Asn Lys Val Ser Tyr Val Tyr Gly His Asp Ala Ser Thr
145                 150                 155                 160

Tyr Gly Ser Gln Asp Asn Leu Asn Pro Leu Ile Asn Glu Ile Ser Lys
                165                 170                 175

Ile Ile Ser Ala Ala Gly Asn Phe Asp Val Lys Glu Glu Arg Ser Ala
            180                 185                 190

Ala Ser Leu Leu Gln Leu Ser Gly Asn Ala Ser Asp Phe Ser Tyr Gly
        195                 200                 205

Arg Asn Ser Ile Thr Leu Thr Ala Ser Ala
    210                 215

<210> 3
<211> 30
<212> DNA
<213> Artificial sequence
<223> Antibody hinge region
<400> 1
cggtccgaag ccttccactc cgcccgggtc 30

<210> 4
<211> 10
<212> PRT
<213> Antibody hinge region
<400> 2
Gly Pro Lys Pro Ser Thr Pro Pro Gly Ser
1               5                   10

<210> 5
<211> 33
<212> DNA
<213> Artificial sequence
<223> BACKHINGE primer
<400> 5
gcgtcgacgt ccggcggtcc gaagccttcc act 33

<210> 6
<211> 44
<212> DNA
<213> Artificial sequence
<223> FORHIS primer
<400> 6
gcgtcgacgc atggtgatgg tgatggtgac cacc 44

<210> 7
<211> 47
<212> DNA

<213> Artificial sequence
<223> VHHA1 primer
<400> 7
ctatgcggcc cagccggcca tggctcaggt gcagctggtg gagtctt          47

<210> 8
<211> 47
<212> DNA
<213> Artificial sequence
<223> VHHASfil primer
<400> 8
cgtcgacgcg gcccccgagg ccgttgagga gacggtgacc tgggtccc          48

## Description

### FIELD OF THE INVENTION

This invention is related to the production of recombinant dimeric proteins by means of the use of a protein expression system based on the *Escherichia coli* hemolysin transport system.

### BACKGROUND OF THE INVENTION

For some time now, the production of fusion proteins comprising bi- or multi- functional recombinant antibody fragments (miniantibodies) has been researched. These fusion proteins have several advantages and may be used for therapeutic or diagnostic purposes. For this reason, different antibody fragment expression systems have been developed. Some of these expression systems are based on the use of *Escherichia coli.*

Different antibody fragments are conventionally chosen and produced in E. *coli* after cloning fragments of the variable (V) and constant (C) regions of immunoglobulins (Ig) in filamentous phage or phagemid vectors [Hoogenboom, H. R. 1997. Designing and optimizing library selection strategies for generating high-affinity antibodies. Trends in Biotechnology. 15:62-70; Hoogenboom, H.R. 2002. Overview of antibody phage-display technology and its applications. Methods Mol Biol. 178:1-37; Winter, G., A.D. Griffiths, R.E. Hawkins, and H.R. Hoogenboom 1994. Making antibodies by phage display technology. Annual Rev. Immunol. 12:433-455]. These fragments reconstruct the antigen binding site of the original antibody which is generally assembled by means of contact of the V domains of the heavy (H) and light (L) chains [Ay, J., T. Keitel, G. Kuttner, H. Wessner, C. Scholz, M. Hahn, and W. Hohne. 2000. Crystal structure of a phage library-derived single-chain Fv fragment complexed with turkey egg-white lysozyme at 2.0 Å resolution. J Mol Bio. 301:239-246]. This is the case of the Fab molecules, which consist of the association of two polypeptides containing the V_{H}-C_{H1} and V_{L}-C_{L} domains and of the single-chain Fv molecules (scFv) in which the V_{H} and V_{L} domains bind in a single polypeptide. The Fab and scFv molecules have significant advantages, such as higher expression levels in E. *coli* and a better distribution and faster clearance when administered in vivo for diagnostic or therapeutic applications [Carter, P. and A.M. Merchant. 1997. Engineering antibodies for imaging and therapy. Current Opinion in Biotechnology. 8:449-454; Marasco, W.A. and S. Dana Jones. 1998. Antibodies for targeted gene therapy: extracellular gene targeting and intracellular expression. Adv Drug Deliv Rev 31:153-170; Yokota, T., D.E. Milenic, M. Whitlow and J. Schlom. 1992. Rapid Tumor penetration of a single-chain Fv and comparison with other immunoglobulin forms. Cancer Res. 52:3402-8].

Antibody fragments based on a single Ig domain have also been produced in E. *coli* [Nuttall, S.D., R.A. Irving and P.J. Hudson. 2000. Immunoglobulin VH domains and beyond: design and selection of single-domain binding and targeting reagents. Curr Pharm Biotechnol. 1:253-63; Riechmann, L. and S. Muyldermans. 1999. Single domain antibodies: comparison of camel VH and camelised human VH domains. J Immunol Methods. 231:25-38; Sheriff, S. and K.L. Constantine. 1996. Redefining the minimal antigen-binding fragment. Nat Struct Biol. 3:733-6]. This progress was carried out as a result of the finding that in camelid species (for example llamas, camels), a proportion of their natural antibodies lack light chains, therefore constructing an antigen binding surface with a single heavy chain V domain (V_{HH}). In addition to the benefits of scFv and Fab molecules, the V_{HH} domains have demonstrated greater stability and solubility and less immunogenicity [Cortez-Retamozo, V., M. Lauwereys, G. Hassanzedeh Gh, M. Gobert, K. Conrath, S. Muyldermans, P. De Baetselier and H. Revets. 2002. Efficient tumor targeting by single-domain antibody fragments of camels. Int J Cancer. 98:456-62; Nuttall, S.D., R.A. Irving and P.J. Hudson. 2002. Immunoglobulin VH domains and beyond: design and selection of single-domain binding and targeting reagents. Curr Pharm Biotechnol. 1:253-63; Riechmann, L. and S. Muyldermans. 1999. Single domain antibodies: comparison of camel VH and camelised human VH domains. J Immunol Methods. 231:25-38]. However, all these antibody fragments lose antigen binding bivalence (or multivalence) shown by whole antibodies. Their monovalent nature is reflected by a decreased functional affinity (avidity) for their corresponding antigens. In order to solve this problem, short oligomerization domains (for example, amphipathic helixes) were obtained by genetic engineering at the C-terminal ends in order to produce bivalent and tetravalent miniantibodies with identical avidity as whole antibodies [Pack, P., M. Kujau, V. Schroeckh, U. Knupfer, R. Wenderoth, D. Riesenberg and A. Plückthun. 1993. Improved bivalent miniantibodies with identical avidity as whole antibodies, produced by high cell density fermentation of *Escherichia coli.* Biotechnology (NY). 11:1271-7; Pack, P., K. Muller, R. Zahn and A. Plückthun. 1995. Tetravalent miniantibodies with high avidity assembling in *Escherichia coli.* J Mol Bio. 246:28-34; Pack, P. and A. Plückthun. 1992. Miniantibodies: use of amphipathic helices to produce functional, flexibly linked dimeric FV fragments with high avidity in *Escherichia coli.* Biochemistry. 31:1579-84; Plückthun, A. and P. Pack. 1997. New protein engineering approaches to multivalent and bispecific antibody fragments. Immunotechnology. 3:83-105; Rheinnecker, M., C. Hardt, L.L. Ilag, P. Kufer, R. Gruber, A. Hoess, A. Lupas, C. Rottenberger, A. Plückthun and P. Pack. 1996. Multivalent antibody fragments with high functional affinity for a tumor-associated carbohydrate antigen. J Immunol. 157:2989-97].

In almost all cases, the monovalent and multivalent antibody fragments have been produced in the periplasmic space in *E. coli,* a signal peptide, which is recognized by the cellular machinery of the general secretion route (Sec), being fused to them at the N-terminal end (N-SP) [Plückthun, A., C. Krebber, U. Krebber, U. Horn, U. Knüpfer, R. Wenderoth, L. Nieba, K. Proba and D. Riesenberg. 1996. Producing antibodies in *Escherichia coli:* from PCR to fermentation, p. 203-252. *In* J. McCafferty and H.R. Hoogenboom (eds), Antibody Engineering: A Practical Approach. IRL Press, Oxford]. An alternative method has recently been disclosed for producing functional scFvs in the *E. coli* extracellular medium which uses the α-hemolysin (Hly) transporter [Fernández, L.A., I. Sola, L. Enjuanes and V. de Lorenzo. 2000. Specific secretion of active single-chain Fv antibodies into the supernatants of *Escherichia coli* cultures by use of the hemolysin system. Appl Environ Microbiol. 66:5024-5029]. This secretion system is independent from the cellular sec genes and consists of two inner membrane (IM) components, HlyB and HlyD, and the outer membrane (OM) pore, TolC, which are assembled in a large protein complex with an internal hydrophilic channel [Gentschev, I., G. Dietrich and W. Goebel. 2002. The E. coli alpha-hemolysin secretion system and its use in vaccine development. Trends Microbiol. 10:39-45; Koronakis, V., C. Andersen and C. Hughes. 2001. Channel-tunnels. Curr Opin Struct Biol. 11:403-7; Koronakis, V., A. Sharff, B. Luisi and C. Hughes. 2000. Crystal structure of the bacterial membrane protein TolC central to multidrug efflux and protein export. Nature. 405:914-919; Thanabalu, T., E. Koronakis, C. Hughes and V. Koronakis. 1998. Substrate-induced assembly of a contiguous channel for protein export from *E. coli:* reversible bridging of an inner-membrane translocase to an outer membrane exit pore. EMBO J. 17:6487-96]. The natural substrate of this system, the α-hemolysin (HlyA) toxin, is expelled through this channel directly from the cytoplasm towards the extracellular medium without a periplasmic intermediate and in an ATP-dependent manner. The signal recognized by the Hly secretion machinery is located at the HlyA C-terminal end. It has been demonstrated that scFv-HlyA hybrids containing an scFv molecule lacking the N-SP bound to the last HlyA of about 23 kDa are secreted in a functional manner and oxidized by the Hly transporter [Fernández, L.A. and V. de Lorenzo. 2001. Formation of disulphide bonds during secretion of proteins through the periplasmic-independent type I pathway. Mol Microbiol. 40:332-46; Fernández, L.A., I. Sola, L. Enjuanes and V. de Lorenzo. 2000. Specific secretion of active single-chain Fv antibodies into the supernatants of *Escherichia coli* cultures by use of the hemolysin system. Appl Environ Microbiol. 66:5024-5029].

On the other hand, dimerization is a property that is frequently desired to be achieved by genetic protein engineering when a binding activity is involved (for example, in protein-DNA or antigen-antibody interactions) since it may intensify their functional affinity (avidity) or create bi-specific molecules [Baxevanis, A.D. and C.R. Vinson. 1993. Interactions of coiled coils in transcription factors: where is the specificity? Curr Opin Genet Dev. 3:278-85; Busch, S.J. and P. Sassone-Corsi. 1990. Dimers, leucine zippers and DNA-building domains. Trends Genet. 6:36-40; Crothers, D.M. and H. Metzger. 1972. The influence of polyvalency on the binding properties of antibodies. Immunochemistry. 9:341-357; Plückthun, A. and P. Pack. 1997. New protein engineering approaches to multivalent and bispecific antibody fragments. Immunotechnology. 3:83-105]. A process for producing dimeric proteins comprising two monomeric fusion proteins in a non-covalent interaction has been disclosed in US patent 5,910,573. The dimeric proteins thus obtained are accumulated within the cell in the periplasmic space without being secreted to the extracellular medium. This leads to a higher toxicity of their expression for the *E. coli* bacteria, inducing a lower yield in cultures (dry cell weight per liter) and making the subsequent purification of the dimeric antibody difficult as the bacteria must be lysed (broken up).

Therefore the need still exists to develop alternative systems for producing dimeric proteins.

### SUMMARY OF THE INVENTION

The invention provides a solution to the existing need based on the development of a DNA construct comprising (i) a nucleotide sequence coding for a product of interest; (ii) a nucleotide sequence coding for a dimerization domain; and (iii) a nucleotide sequence coding for α-hemolysin (HlyA) of *Escherichia coli* or for a fragment of said protein comprising the recognition signal of the E. *coli* hemolysin (Hly) transport system secretion mechanism. Dimeric proteins are obtained in the medium by means of the use of said protein expression and secretion system. The efficacy of said secretion system has been demonstrated by means of the production of high avidity miniantibodies derived from camel V_{HH} antibodies (Example 1).

The E. *coli* hemolysin translocator had previously been used for secreting heterologous polypeptides, especially pathogen antigens and toxins, as well as for secreting scFv recombinant antibodies. However, the results now obtained clearly show that the incorporation of an autodimerization amphipathic helix at the C-HlyA N-terminal end does not interfere with Hly secretion and allows the dimerization of the secreted polypeptide. The dimerization likewise intensifies the avidity of the binding of the secreted polypeptide derivative of C-HlyA. Furthermore, it may also have other applications, such as the molecular association of several antigens and/or adjuvants produced by live bacterial strains, or the combination of different biological activities for generating bispecific molecules (for example, antigen binding and complement recruiting).

Therefore, one aspect of this invention is related to a DNA construct comprising (i) a nucleotide sequence coding for a product of interest; (ii) a nucleotide sequence coding for a dimerization domain; and (iii) a nucleotide sequence coding for *Escherichia coli* α-hemolysin (HlyA) or for a fragment of said protein comprising the recognition signal of the *E. coli* hemolysin (Hly) transport system secretion mechanism.

In another aspect, the invention relates to an expression cassette comprising said DNA construct operatively bound to an expression control sequence.

In another aspect, the invention relates to a bacteria comprising at least one DNA construct or at least one expression cassette.

In another aspect, the invention relates to a method for producing a product of interest in the form of a dimeric fusion protein, which comprises growing said bacteria under conditions allowing the production and excretion to the culture medium of said product of interest in the form of a dimeric fusion protein.

In a preferred aspect of the invention, the latter is related to a method for producing a heterodimeric fusion protein comprising two products of interest.

In another aspect, the invention relates to a dimeric fusion protein that can be obtained by expression of at least one nucleic acid sequence contained in at least one DNA construct.

### BRIEF DESCRIPTION OF THE FIGURES

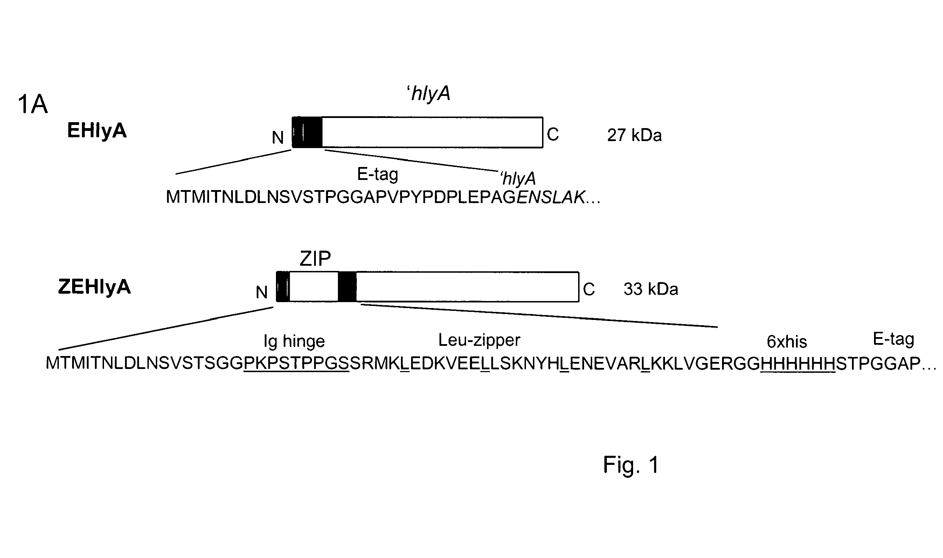

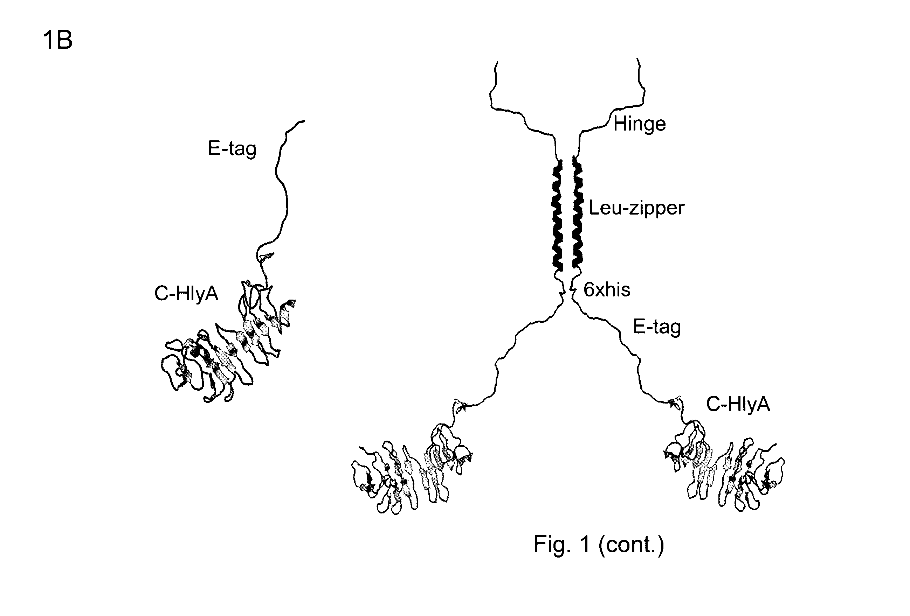

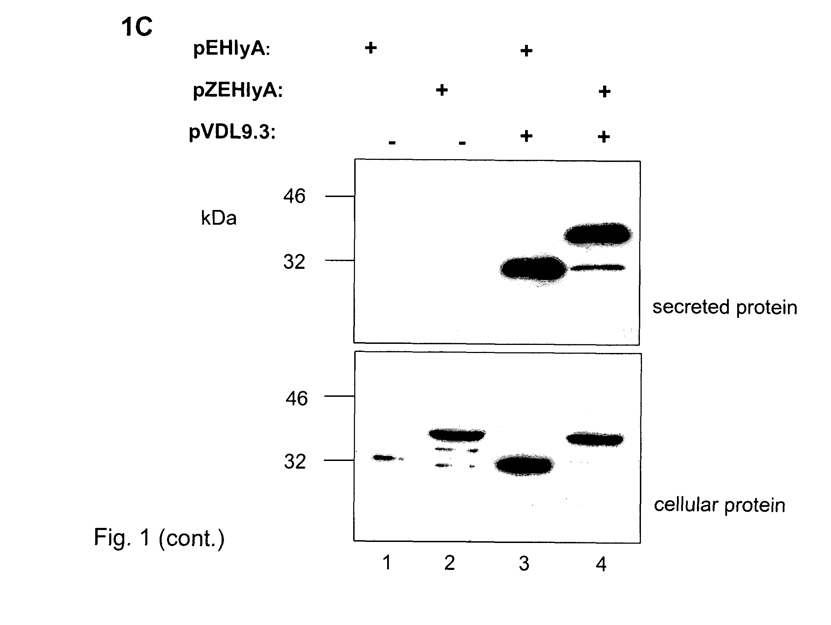

Figure 1 shows the secretion of the C-HlyA polypeptide containing the ZIP domain. Figure 1A shows a schematic representation of the structure of the polypeptides EHlyA and ZEHlyA containing the 23 kDa *('hlyA)* secretion signal of the *E. coli* Hly transporter tagged with the E epitope. The mass of said polypeptides (in kDa), deduced from its amino acid sequence, is shown to the right. The composition of the ZIP domain (Ig hinge, leucine zipper, 6xhis tag) is indicated. The amino acid sequence of the N-terminal region of both polypeptides EHlyA and ZEHlyA is also shown. Figure 1B shows a schematic representation of the polypeptide C-HlyA (monomeric) tagged with the E epitope and of the C-HlyA polypeptide (dimeric) tagged with the E epitope and containing the ZIP domain (Ig hinge, leucine zipper, 6xhis tag). Figure 1C shows the result of the immunoblotting carried out with a POD-labeled anti-E monoclonal antibody of the secreted (S) and cell (C) proteins produced after the 4 hour induction with 0.3 IPTG mM of E. *coli* HB2151 cell cultures, grown at 37°C, containing the plasmid pVDL9.3 (which codes for HlyB and HlyD) and one of the indicated plasmids, pEHlyA or pZEHlyA. The proteins loaded in each lane represent those found in about 5 µl of the supernatants (S) of the culture and those of the E. *coli* cells (C) present in about 100 µl of the same cultures (OD₆₀₀ₙₘ about 2).

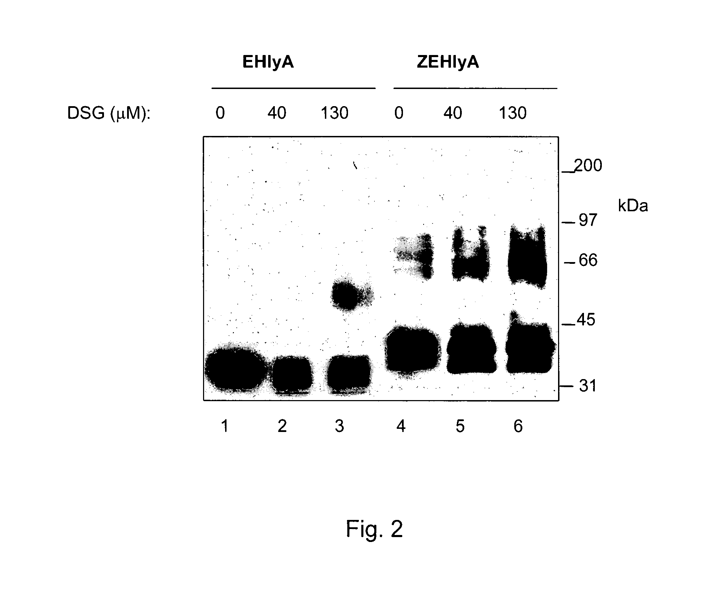

Figure 2 shows the cross-linking of the secreted C-HlyA polypeptides with disuccinimidyl glutarate (DSG). The secreted EHlyA and ZEHlyA polypeptides (about 10 µg/ml in PBS) were incubated with DSG at the indicated concentrations and subjected to denaturing SDS-PAGE and to immunoblotting with POD-labeled anti-E monoclonal antibody (see the Materials and Methods section in Example 1 for more details). As it is shown, ZEHlyA was cross-linked with DSG forming a protein band in SDS-PAGE of about 66 kDa, about twice the size of its monomer.

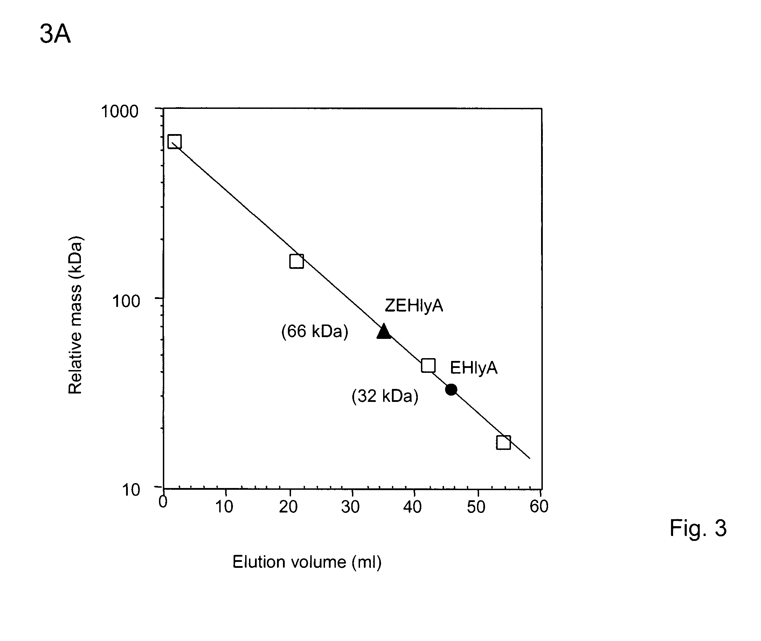

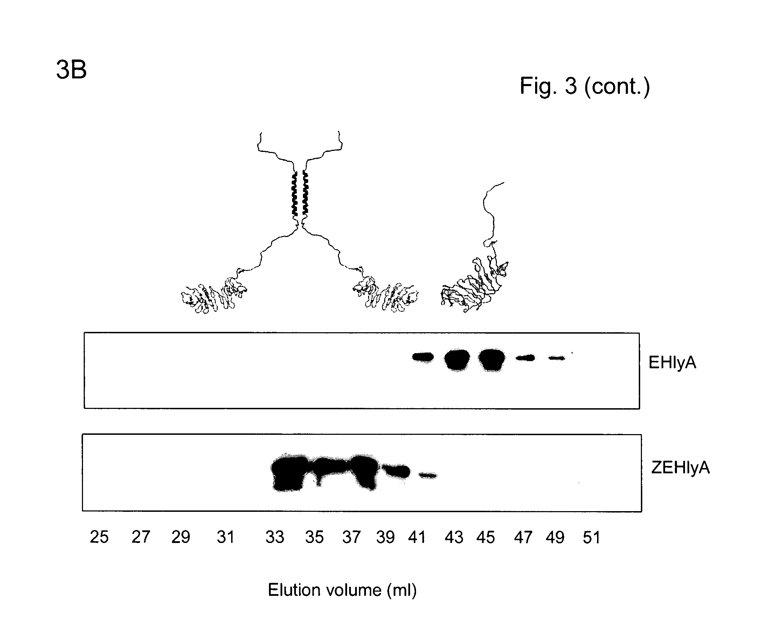

Figure 3 shows the results of the gel filtration chromatography of the monomeric and dimeric C-HlyA polypeptides. Figure 3A shows a graph representing the elution volume of the EHlyA (circle) and ZEHlyA (triangle) polypeptides separated by gel filtration chromatography (see the Materials and Methods section in Example 1 for more details), together with known mass protein standards (squares). The mass standards used were thyroglobulin (Mr 670,000), bovine gamma globulin (Mr 158,000), chicken ovalbumin (Mr 44,000) and equine myoglobin (Mr 17.000). The presence of EHlyA or ZEHlyA in the eluted fractions was determined by immunoblotting with POD-labeled anti-E monoclonal antibody. Figure 3B shows the result of the immunoblotting carried out with a POD-labeled anti-E monoclonal antibody of the EHlyA and ZEHlyA polypeptides. A schematic representation of EHlyA (monomeric) and of ZEHlyA (dimeric) is shown in the upper portion.

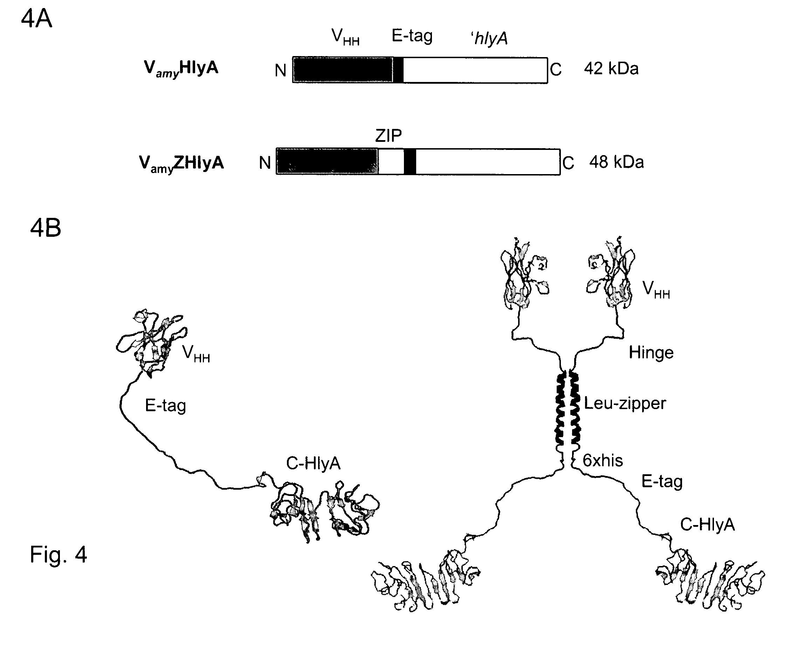

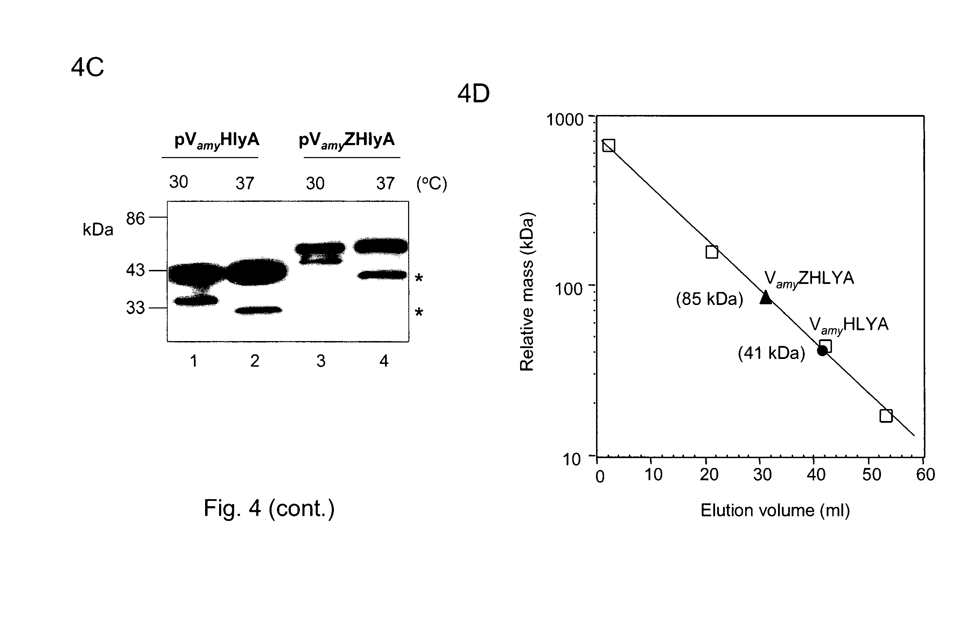

Figure 4 shows the secretion of monomeric V_{HH}-HlyA polypeptides (V_{amy}-HlyA) and dimeric V_{HH} -HlyA polypeptides (V_{amy-}ZHlyA). Figure 4A shows a schematic representation of the structure of the V_{amy}-HlyA and V_{amy}-ZHlyA polypeptides containing the 23 kDa *('hylA)* secretion signal of the E. *coli* Hly transporter tagged with the E epitope. The mass of said polypeptides (in kDa), deduced from their amino acid sequence, is shown to the right. Figure 4B shows a schematic representation of the V_{*amy*}-HlyA polypeptide (monomeric) tagged with the E epitope and of the V_{*amy*}-ZHlyA polypeptide (dimeric) tagged with the E epitope and containing the ZIP domain (Ig hinge, leucine zipper, 6xhis tag). Figure 4C shows the results of a Western blot, clearly showing the secretion of protein hybrids having V_{HH} and -EHlyA or -ZEHlyA domains. The *E. coli* HB2151 cells (pVDL9.3) incorporating one of the indicated plasmids (pV_{*amy*}HlyA or pV_{*amy*}ZHlyA) were induced with IPTG (see the Materials and Methods section in Example 1 for more details) at the indicated temperature, and the presence of secreted polypeptides tagged with the E epitope in supernatants of the culture was determined by immunoblotting with POD-labeled anti-E monoclonal antibody. The full length V_{*amy*}HlyA and V_{*amy*}ZHlyA polypeptides were detected in supernatants of the culture, together with some proteolytic fragments derived therefrom. Figure 4D shows a graph of the elution volume of the V_{*amy*}HlyA (circle) and V_{*amy*}ZHlyA (triangle) polypeptides separated by gel filtration chromatography, together with known mass protein standards (squares) and detected with immunoblotting with POD-labeled anti-E monoclonal antibody. The mass standards used were the same as those used in relation to Figure 3.

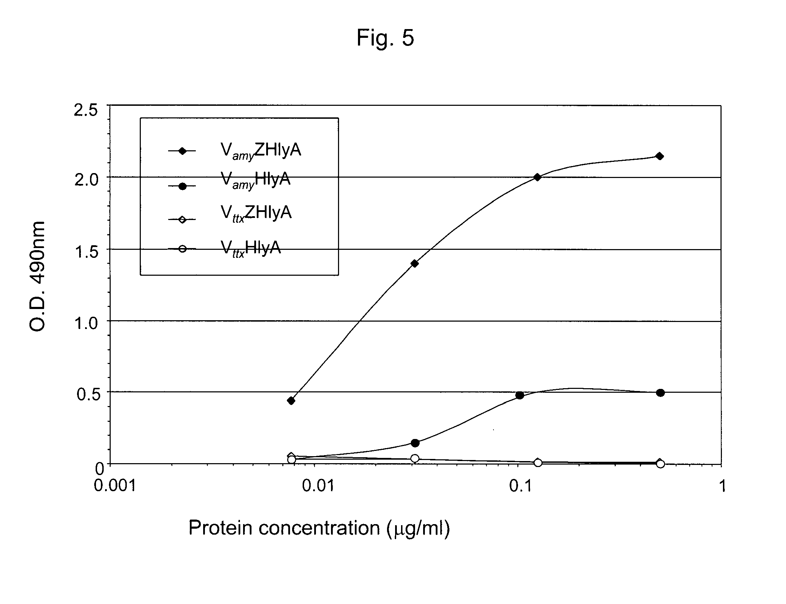

Figure 5 shows a graph illustrating the binding activity of the monomeric and dimeric V_{HH}-HlyA polypeptides. The α-amylase binding by means of the V_{*amy*}HlyA and V_{*am*y}ZHlyA polypeptides at the indicated concentrations was determined by means of ELISA (see the Materials and Methods section in Example 1 for more details). The bound miniantibodies tagged with the E epitope were detected with POD-labeled anti-E monoclonal antibody and O.D. reading at 490 nm. V_{*ttx*}HlyA and V_{*ttx*}ZHlyA polypeptides were used as specificity controls. The prior binding to an unrelated control antigen (ovalbumin) has been subtracted (OD₄₉₀ₙₘ ≤ 0.05). The data shown is the mean of the triplicates of each point. Two further independent experiments were performed, which showed similar values as those shown in the figure.

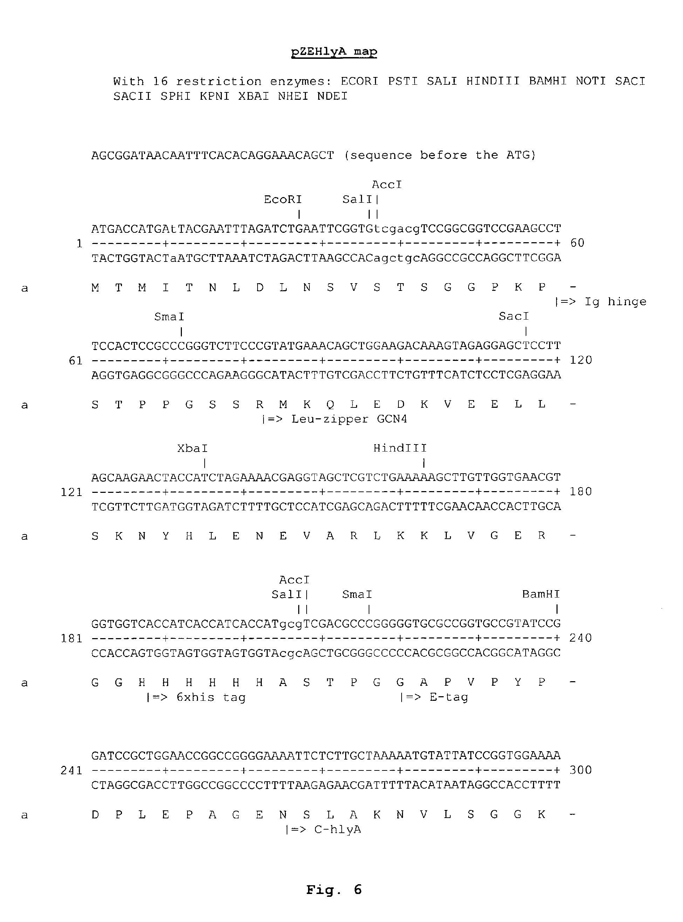

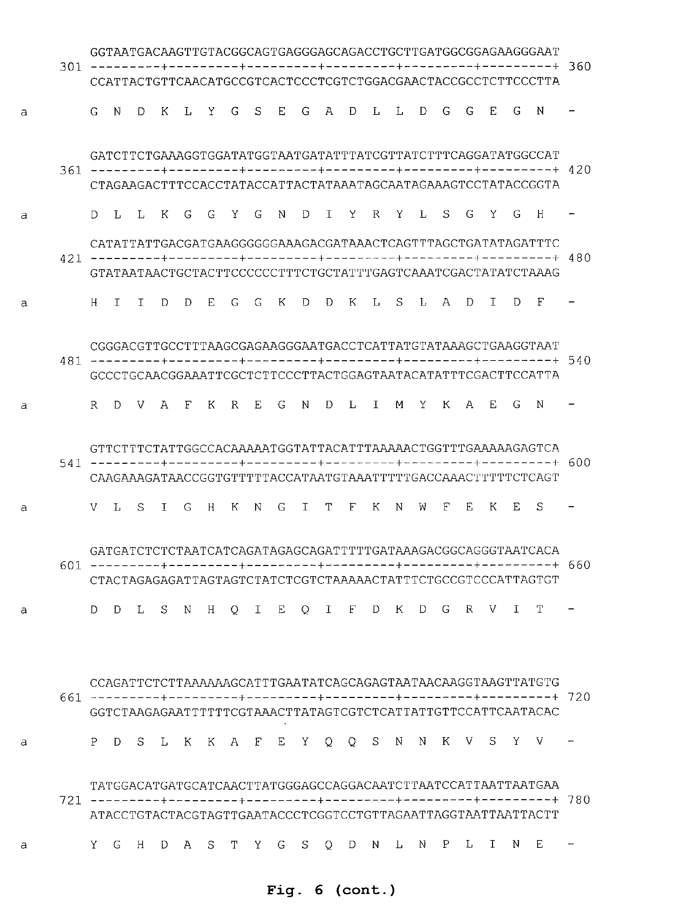

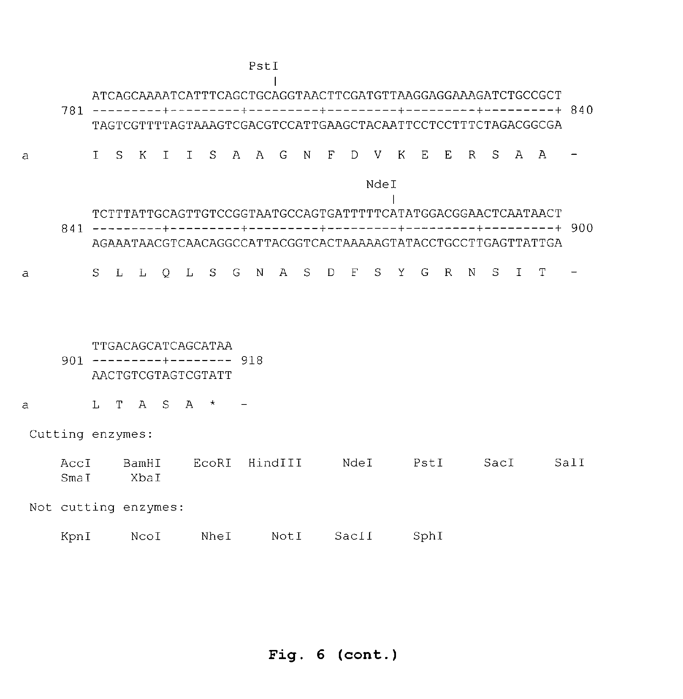

Figure 6 shows the map of the plasmid pZEHlyA.

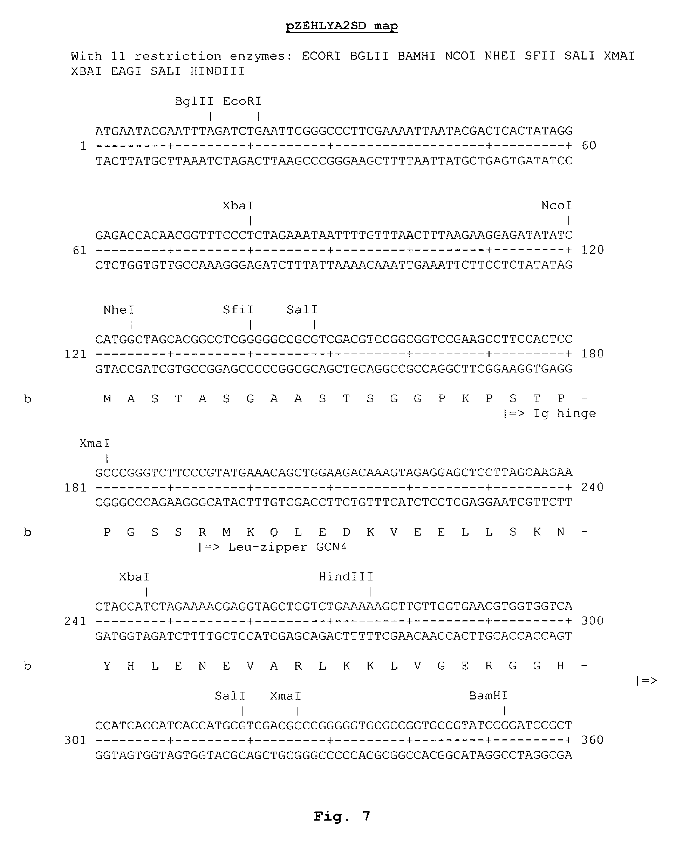

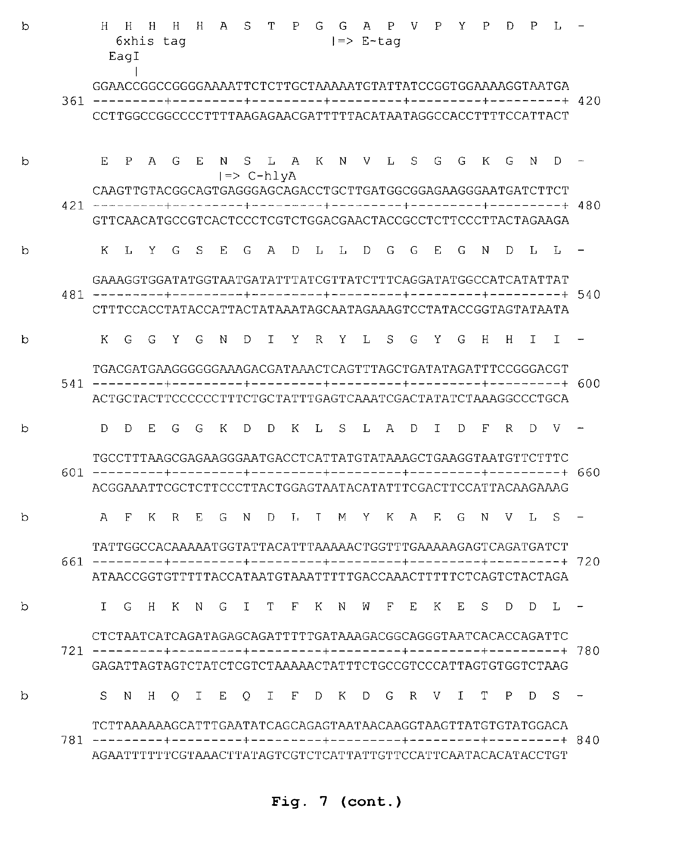

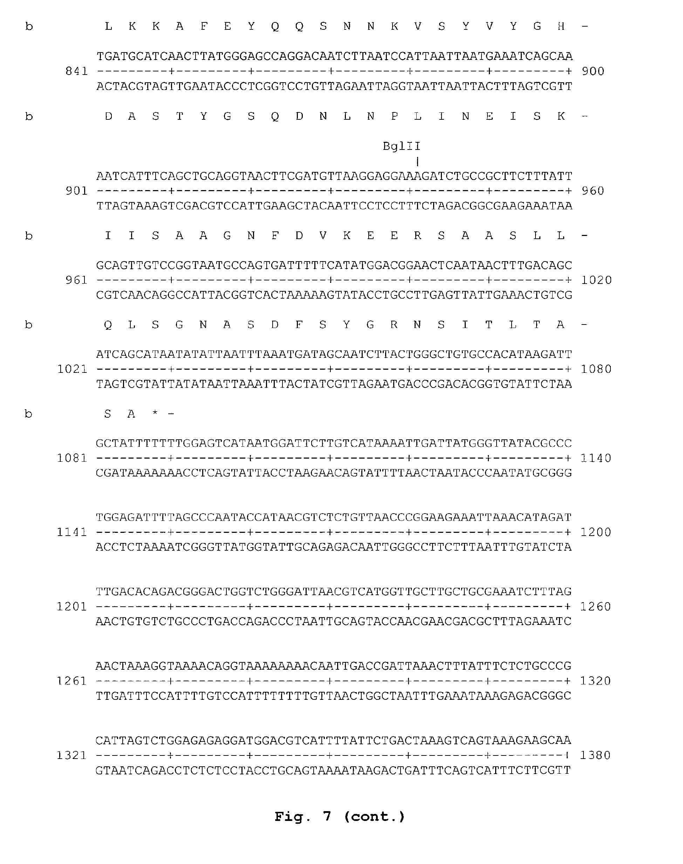

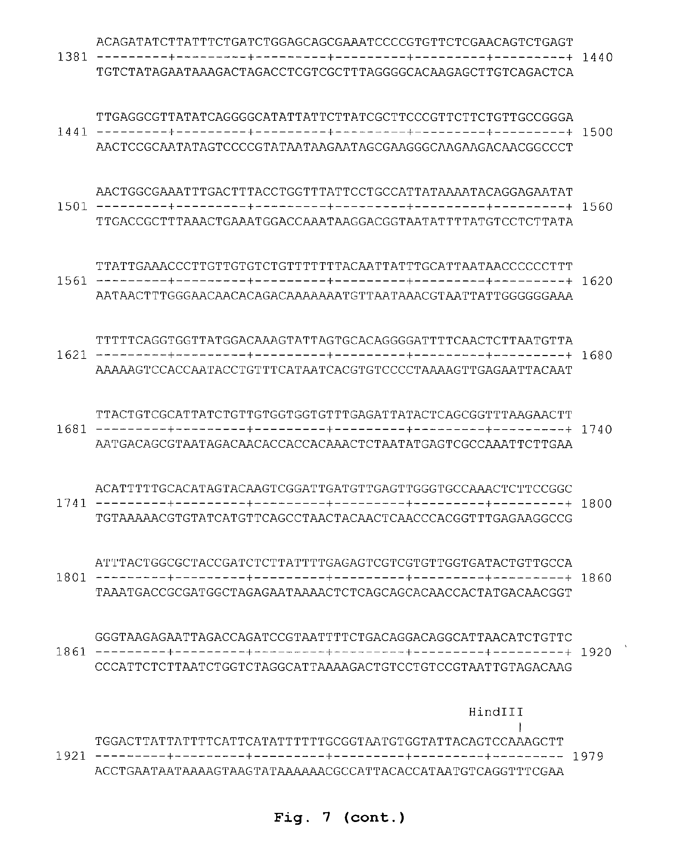

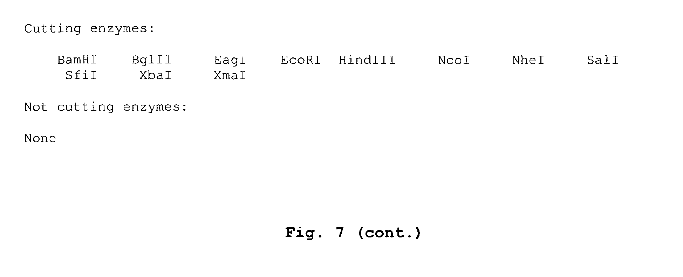

Figure 7 shows the map of the plasmid pZEHlyA2-SD.

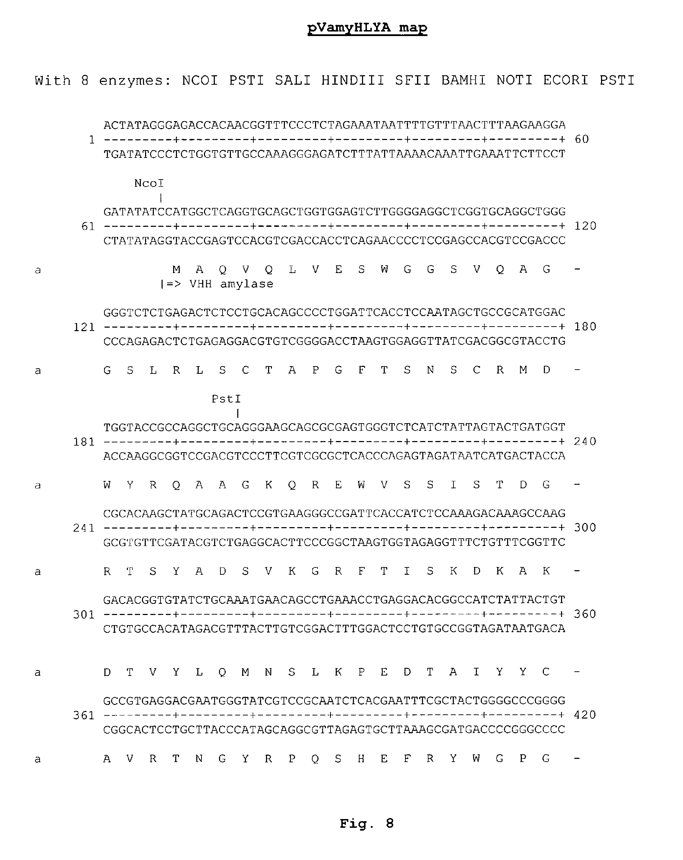

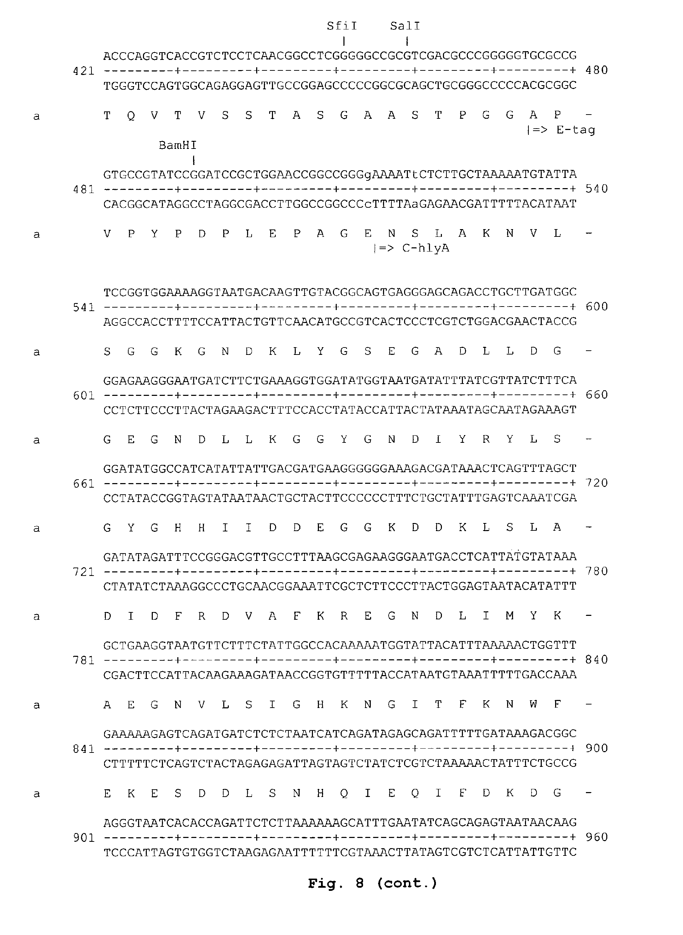

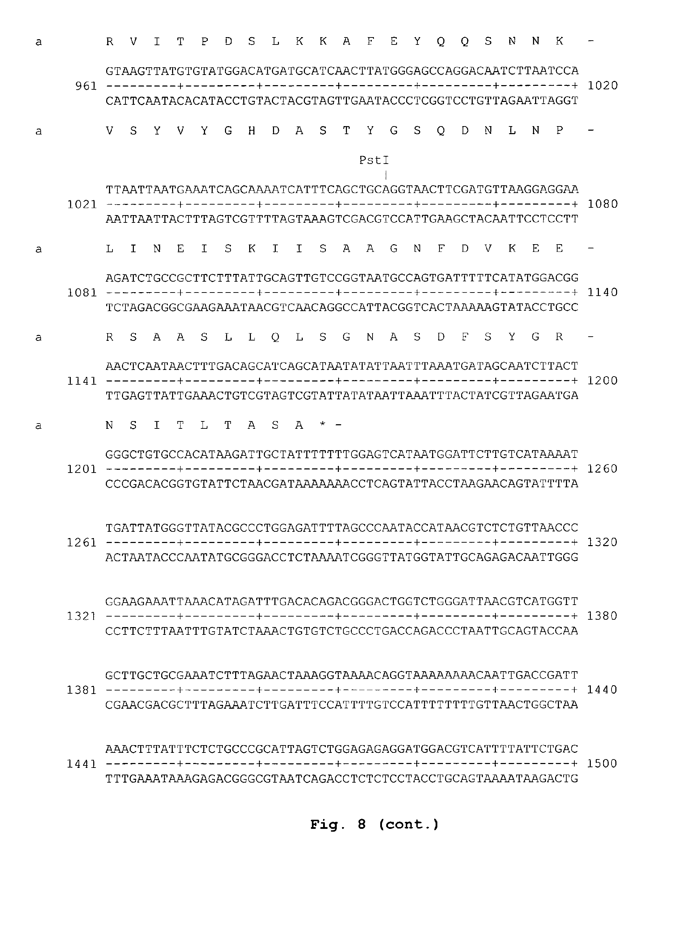

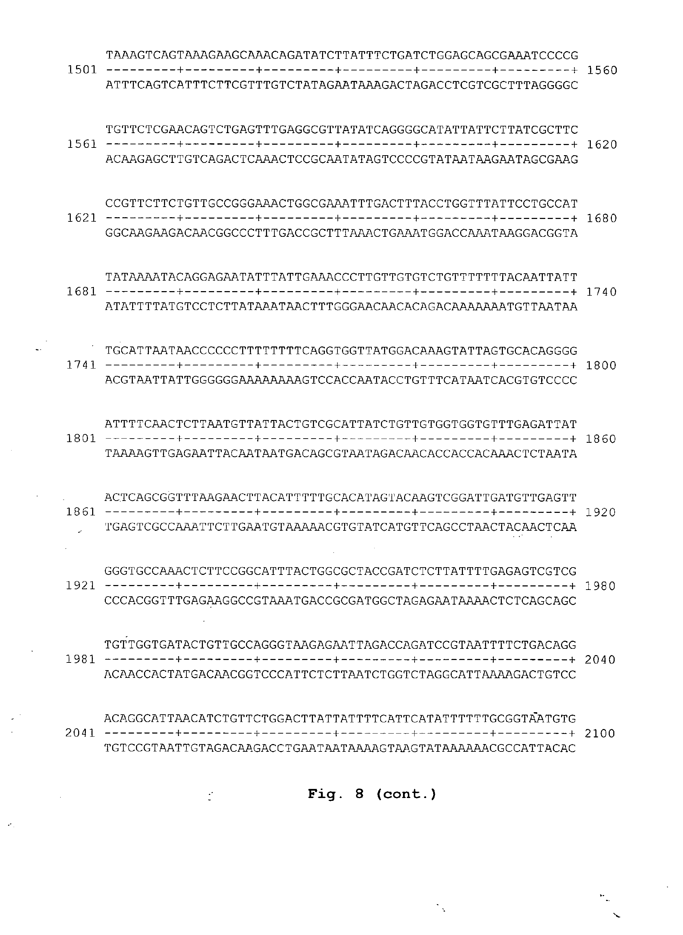

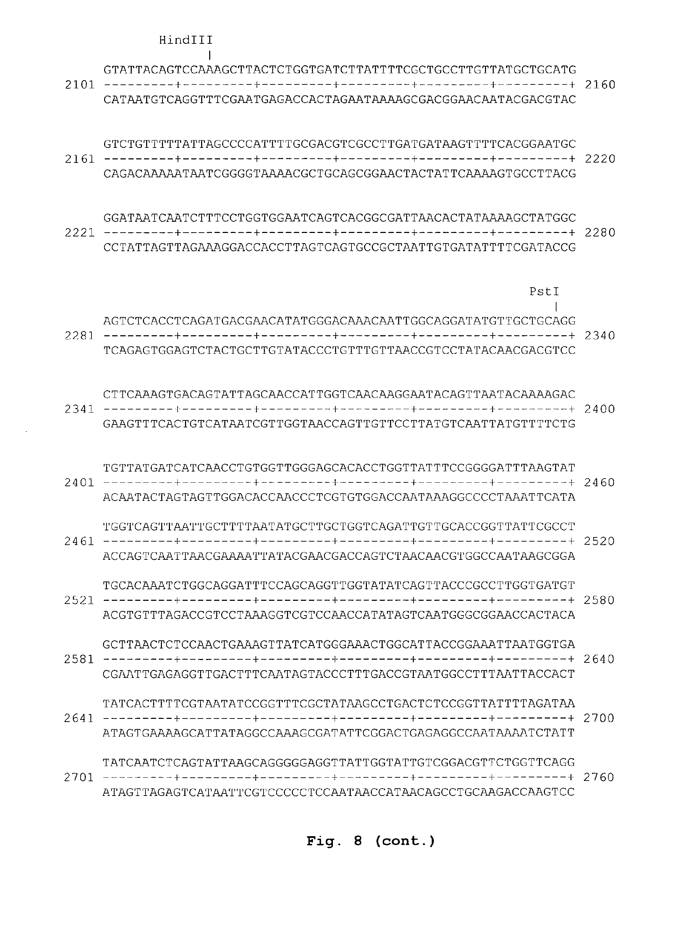

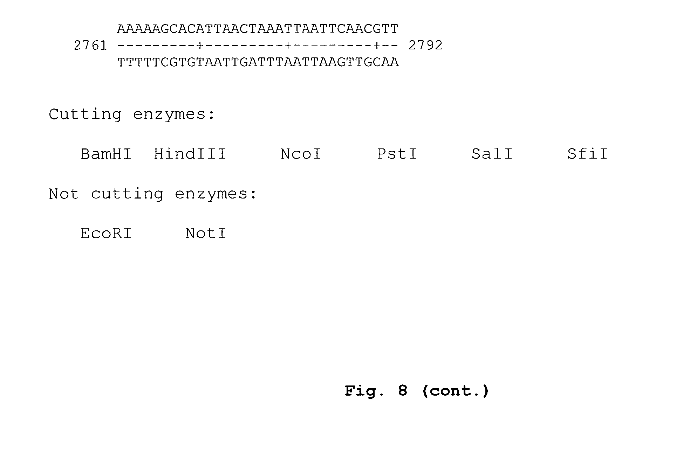

Figure 8 shows the map of the plasmid pV_{*amy*}HlyA.

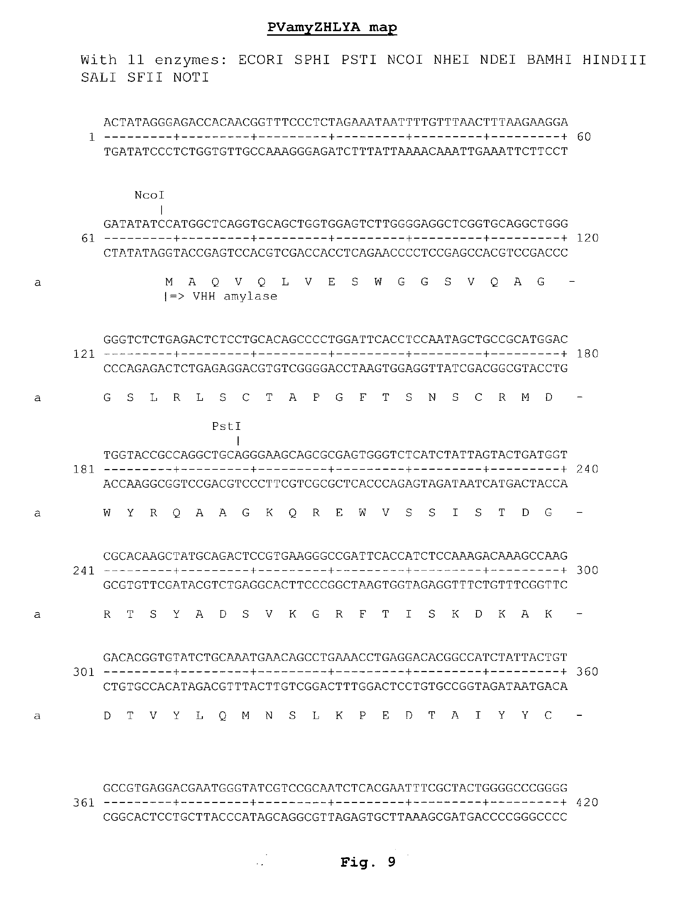

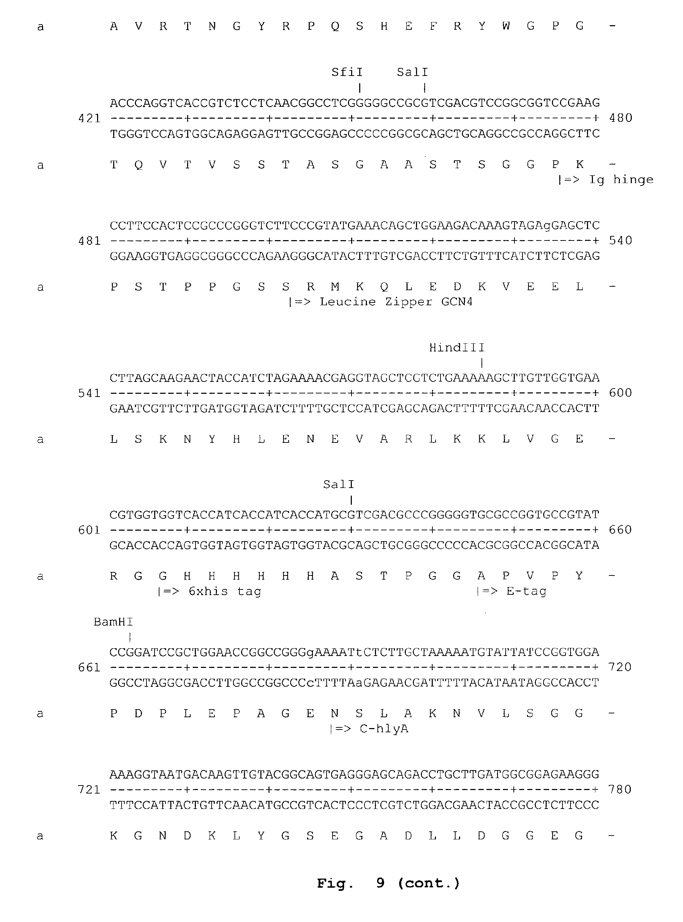

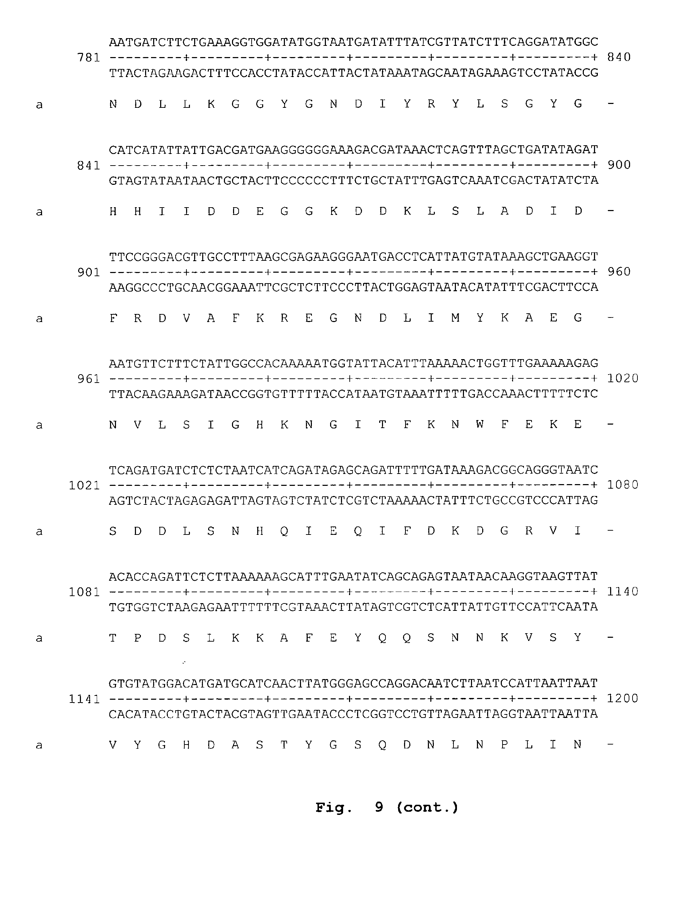

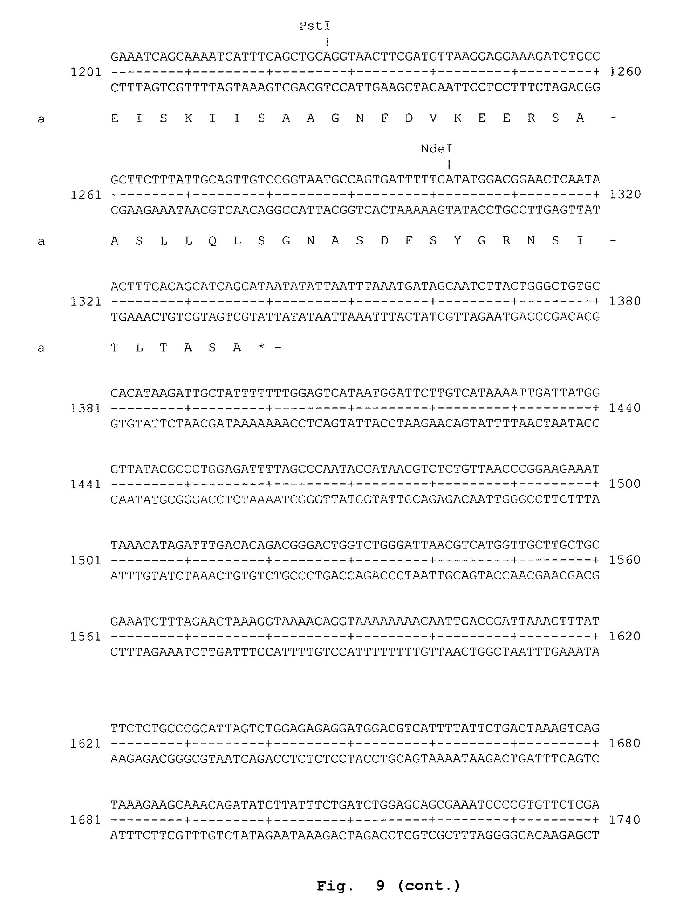

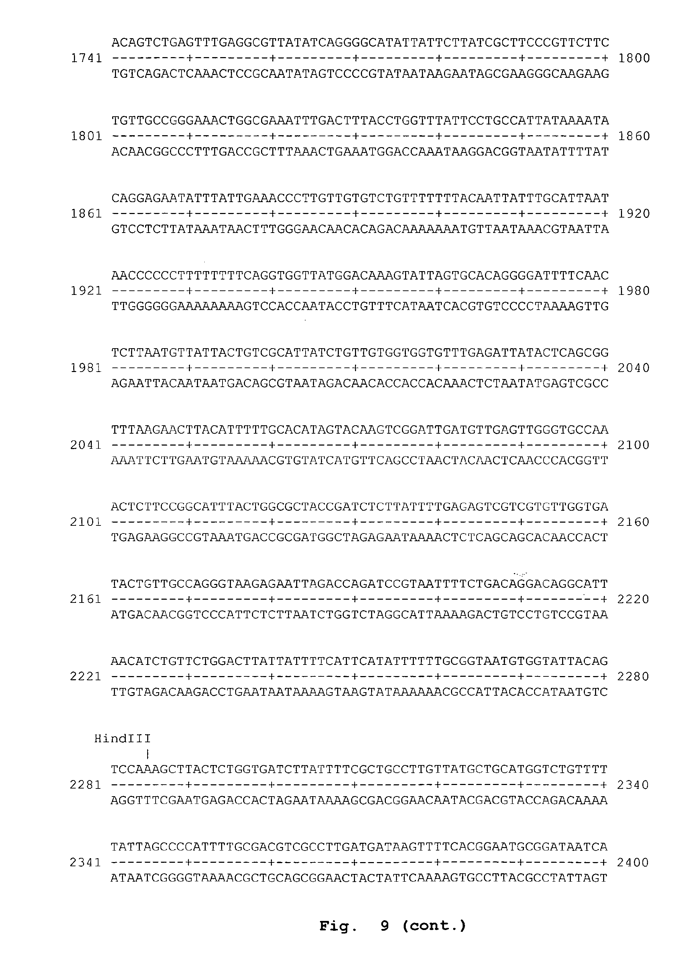

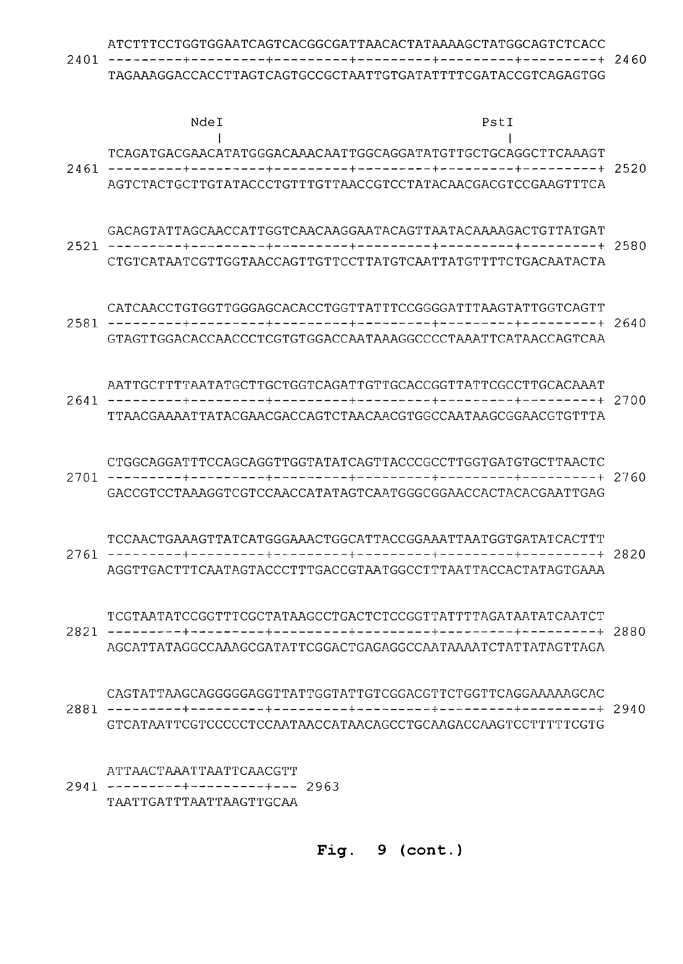

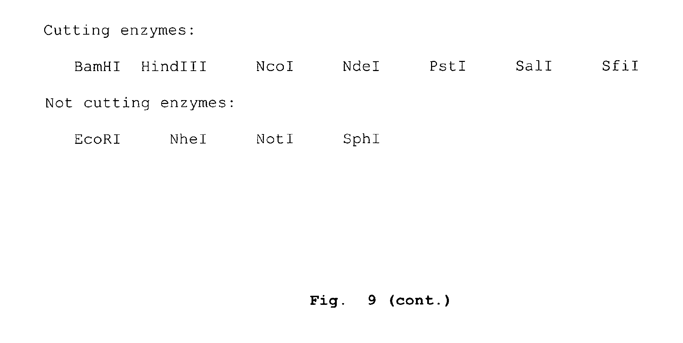

Figure 9 shows the map of the plasmid pV_{*amy*}ZHlyA.

### DETAILED DESCRIPTION OF THE INVENTION

In one aspect, the invention provides a DNA construct, hereinafter DNA construct of the invention, comprising:
a) a first nucleic acid sequence containing the nucleotide sequence coding for a product of interest;
b) a second nucleic acid sequence containing the nucleotide sequence coding for a dimerization domain; and
c) a third nucleic acid sequence containing the nucleotide sequence coding for E. *coli* α-hemolysin (HlyA) or for a fragment of said protein comprising the recognition signal of the *E. coli* Hly transport system secretion mechanism, or a nucleotide sequence coding for a homologous gene, or a nucleotide sequence coding for a natural or artificial variant of HlyA or of a fragment thereof comprising the recognition signal of the E. *coli* Hly transport system secretion mechanism;
wherein the 3' end of said first nucleic acid sequence is bound to the 5' end of said second nucleic acid sequence, and the 3' end of said second nucleic acid sequence is bound to the 5' end of said third nucleic acid sequence.

The first nucleic acid sequence contains the nucleotide sequence coding for a product of interest (gene of interest). The product of interest may be eukaryotic, prokaryotic, viral, etc. Practically any peptide or protein susceptible to being recombinantly expressed can be used in the DNA construct of the invention, for example enzymes, enzymatic inhibitors, hormones, molecules involved in cell adhesion and/or signaling, molecules involved in detection or labeling, and molecules made up of domains, for example immunoglobulins, etc. As an illustration, said product of interest can be an immunogenic antigen, such as a protein or an antigen fragment thereof, of a pathogen, for example, from a viral, bacterial, parasitic pathogen, etc., which may cause infections in human beings and animals; a therapeutic agent, for example, a tumor-specific antigen, an auto-immune disease antigen, etc.; or an immunoregulating molecule, for example, growth factors, cytokines, such as interleukins, interferons, etc. In a particular embodiment, said product of interest is a miniantibody, defining miniantibodies or recombinant antibodies as fragments derived from the antibodies constructed by recombinant DNA technology and which, despite their smaller size, conserve the capacity to bind to the antigen since they maintain the immunoglobulin variable domains where the antigen binding areas are located.

The second nucleic acid sequence contains the nucleotide sequence coding for a dimerization domain. A dimerization domain is a peptide sequence that promotes dimerization in proteins that contain it. Virtually any dimerization domain can be used in the DNA construct of the invention, for example peptide helixes containing at least one helix, or a structure formed by a helix, a coil and another helix, etc., coiled coil structures, and generally any peptide sequence promoting dimerization in proteins that contain it. In a particular embodiment, said dimerization domain comprises the leucine zipper of the yeast transcription factor GCN4.

The third nucleic acid sequence comprises the nucleotide sequence coding for *E. coli* α-hemolysin (HlyA) or for a fragment of said protein comprising the recognition signal of the *E*. *coli* Hly transport system secretion mechanism, or a nucleotide sequence coding for a homologous gene, or a nucleotide sequence coding for a natural or artificial variant of HlyA or of a fragment thereof comprising the recognition signal of the *E.* coli Hly transport system secretion mechanism. The recognition signal of the E. *coli* Hly transport system secretion mechanism seems to be comprised within the carboxyl terminal end (C-terminal), specifically within the last 60 amino acids of HlyA. The amino acid and nucleotide sequence of E. *coli* HlyA can be obtained from GeneBank, access number M10133, where the nucleotide sequence of HlyB and HlyD amino acids can also be obtained. In a particular embodiment, said third nucleic acid sequence is made up of the nucleic acid sequence coding for E. coli HlyA. In another particular embodiment said third nucleotide sequence comprises a fragment of the E. *coli* HlyA containing the recognition signal of the E. *coli* Hly transport system secretion mechanism, such as a nucleotide sequence coding for the last 60 C-terminal end amino acids of *E. coli* HlyA. In this case, said third nucleic acid sequence is made up of, or comprises, the nucleic acid sequence coding for the last 60 amino acids of the C-terminal end of E. *coli* HlyA.

In a specific embodiment of the invention, said third nucleic acid sequence contains the nucleotide sequence identified as SEQ ID NO: 1 coding for a peptide of about 23 kDa of the carboxyl terminal end of *E. coli* HlyA, the amino acid sequence of which is shown in SEQ ID NO: 2.

Generally, the dimerization domain is not directly fused to the gene encoding the product of interest, but it is advantageous to introduce a spacer (flexible) peptide between the end of the gene coding for the product of interest and the beginning of the dimerization domain. Therefore, if so desired, the DNA construct of the invention can further contain a fourth nucleic acid sequence coding for a spacer peptide located between said first and second nucleic acid sequences, wherein the 5' end of said fourth nucleic acid sequence is bound to the 3' end of said first nucleic acid sequence, and the 3' end of said fourth nucleic acid sequence is bound to the 5' end of said second nucleic acid sequence. In this manner the coding sequence of the product of interest is bound to the dimerization domain by means of a spacer peptide. Advantageously, said spacer peptide is a peptide with structural flexibility. Virtually any peptide with structural flexibility can be used. As an example, said flexible peptide could contain repetitions of amino acid residues, such as Gly-Gly-Gly-Ser, or any other suitable repetition of amino acid residues, or else the hinge region of an antibody. In a particular embodiment, said flexible spacer peptide comprises the hinge region of an antibody and the DNA construct of the invention contains the coding sequence for said flexible peptide. In a specific embodiment of the invention, said fourth nucleic acid sequence contains the nucleotide sequence identified as SEQ ID NO: 3 coding for a 10-amino acid peptide comprising the hinge region of an antibody the amino acid sequence of which is shown in SEQ ID NO: 4.

To facilitate the isolation and purification of the peptide or fusion protein obtained by means of the present invention, the DNA construct of the invention may contain, if so desired, a nucleic acid sequence coding for a peptide susceptible of being used for purposes of isolating or purifying the peptide or fusion protein. Therefore, in a particular embodiment, the DNA construct of the invention contains, if so desired, a fifth nucleic acid sequence coding for a peptide susceptible of being used for isolation or purification purposes.

Virtually any peptide or peptide sequence which allows the isolation or purification of the peptide or fusion protein can be used, for example a polyhistidine sequence, a peptide sequence recognized by a monoclonal antibody and which can be useful for purifying the resulting fusion protein by means of immunoaffinity chromatography, for example tag peptides such as c-myc, HA, E, FLAG, etc. [Using Antibodies: a laboratory manual. Ed Harlow and David Lane (1999). Cold Spring Harbor Laboratory Press. New Cork. Chapter: Tagging proteins. pp. 347-377] and generally any other sequence recognized by an antibody.

Said fifth nucleic acid sequence may be located in any position of the DNA construct of the invention except in the region corresponding to the HlyA C-terminal end since, in this case, it would break the secretion signal. As an example, said fifth nucleic acid sequence could be located between said second and third nucleic acid sequences, wherein the 5' end of said fifth nucleic acid sequence is bound to the 3' end of said second nucleic acid sequence, and the 3' end of said fifth nucleic acid sequence is bound to the 5' end of said third nucleic acid sequence. Alternatively, said fifth nucleic acid sequence could be located in the region corresponding to the N-terminal end of the resulting fusion protein or between the product of interest and the dimerization domain.

In order to facilitate recognition of the obtained peptide or fusion protein, the DNA construct of the invention may also contain, if so desired, a sixth nucleic acid sequence coding for a peptide susceptible of being used for recognition purposes.

Virtually any peptide or peptide sequence which allows the recognition of the peptide or fusion protein can be used, for example a peptide sequence recognized by a monoclonal antibody and which can be useful for recognizing the resulting fusion protein by means of immunodetection techniques, for example tag peptides such as c-myc, HA, E, FLAG, and generally any other sequence recognized by an antibody.

Said sixth nucleic acid sequence may be located in any position of the DNA construct of the invention except in the region corresponding to the HlyA C-terminal end to prevent the secretion signal from being broken. As an example, said sixth nucleic acid sequence could be located between said second and third nucleic acid sequences, wherein the 5' end of said sixth nucleic acid sequence is bound to the 3' end of said second nucleic acid sequence, and the 3' end of said sixth nucleic acid sequence is bound to the 5' end of said third nucleic acid sequence. Alternatively, said sixth nucleic acid sequence could be located in the region corresponding to the N-terminal end of the resulting fusion protein or between the product of interest and the dimerization domain.

Said fifth and sixth nucleic acid sequences could be separated from one another. Alternatively, in a particular embodiment, said fifth and sixth nucleic acid sequences may be joined together. In this case, as an example, said sixth nucleic acid sequence coding for a peptide susceptible of being used for recognition purposes may be located between said third and fifth nucleic acid sequences, wherein the 5' end of said sixth nucleic acid sequence is bound to the 3' end of said fifth nucleic acid sequence, and the 3' end of said sixth nucleic acid sequence is bound to the 5' end of said third nucleic acid sequence. Alternatively, said sequences may be bound together in reverse order, in which case, said sixth nucleic acid sequence coding for a peptide susceptible of being used for recognition purposes is located between said second and fifth nucleic acid sequences, wherein the 3' end of said sixth nucleic acid sequence is bound to the 5' end of said fifth nucleic acid sequence, and the 5' end of said sixth nucleic acid sequence is bound to the 3' end of said second nucleic acid sequence.

If so desired, the DNA construct of the invention may further contain a nucleotide sequence coding for an amino acid sequence susceptible of being cleaved specifically by enzymatic or chemical means for the purpose of releasing the dimeric protein of interest once the fusion protein is isolated. In this case, the DNA construct of the invention may further include a seventh nucleic acid sequence comprising a nucleotide sequence coding for an amino acid sequence susceptible of being cleaved specifically by enzymatic or chemical means. Virtually any amino acid sequence susceptible of being cleaved specifically by enzymatic or chemical means can be used. In a particular embodiment, said seventh nucleic acid sequence comprises a nucleotide sequence coding for a protease recognition site, for example an enterokinase, endoprotease Arg-C, endoprotease Glu-C, endoprotease Lys-C, coagulation factor Xa, and the like. In another particular embodiment said seventh nucleic acid sequence comprises a nucleotide sequence coding for a site susceptible of being cleaved specifically by a chemical reagent such as, for example, cyanogen bromide, which cleaves methionine residues, or any other suitable chemical reagent.

Said seventh nucleic acid sequence is generally located after the 3' end of said second nucleic acid sequence, in any position between the second and third nucleic acid, such that the dimeric protein of interest can be cleaved off by enzymatic or chemical means.

The DNA construct of the invention may be obtained by means of the use of techniques widely known in the state of the art [Sambrook et al., "Molecular cloning, a Laboratory Manual", 2^{nd} ed., Cold Spring Harbor Laboratory Press, N.Y., 1989, Vol 1-3]. Said DNA construct of the invention may incorporate an operatively bound expression regulating sequence, thus forming an expression cassette.

Therefore in another aspect, the invention provides at least one expression cassette comprising at least one DNA construct of the invention operatively bound to an expression control sequence. The control sequences are sequences controlling and regulating transcription and, as the case may be, the translation of the product of interest, and they include promoter sequences *(pT7, plac, pBAD, ptet,* etc.), coding sequences for transcriptional regulators *(lacI, tetR, araC,* etc.), ribosome binding sequences (RBS), and/or transcription terminating sequences *(tlt2,* etc.), etc. In a particular embodiment said expression control sequence is functional in bacteria, particularly in gram-negative bacteria.

The DNA construct of the invention, or the expression cassette provided by this invention, may be inserted in a suitable vector. Therefore, in another aspect, the invention is related to a vector, such as an expression vector, comprising at least one DNA construct or at least one expression cassette. The choice of the vector will depend on the host cell in which it will subsequently be introduced. As an example, the vector in which said DNA sequence is introduced may be a plasmid or a vector which, when introduced in a host cell, is integrated in the genome of said cell or not. Obtaining said vector may be carried out by conventional methods known by persons skilled in the art [Sambrook et al., 1989, cited above].

Advantageously, said vector further comprises a label or gene coding for a motif or for a phenotype allowing the selection of the host cell transformed with said expression cassette. Illustrative examples of said labels which could be present in the expression cassette of the invention include genes resistant to antibiotics, for example ampicillin, tetracycline, kanamycin, chloramphenicol, spectinomycin, etc., or genes resistant to toxic compounds (tellurite, mercury, etc.).

In another aspect the invention is related to a bacteria, particularly a gram-negative bacteria, comprising at least one DNA construct of the invention or at least one expression cassette of the invention, or at least one vector of the invention, hereinafter bacteria of the invention. Said bacteria must have the E. *coli* hemolysin (Hly) exporter system, to which end, if it does not have it naturally, said system must be provided to the bacteria, transforming it with a vector containing the HlyB and HlyD genes, for example the plasmid pVDL9.3 [Fernández, L.A. et al., Applied and Environmental Microbiology, Nov. 2000, 5024-5029]. Virtually any gram-negative bacteria, for example *E. coli, Salmonella typhimurium, Pseudomonas aeruginosa, Pseudomonas putida,* etc., can be converted with the DNA construct of the invention or with the expression cassette of the invention. To that end, the promoting, regulating, labeling signals, and replication origins must be optimized for each bacterial species. In a particular embodiment, said gram-negative bacteria is *Escherichia coli.*

The DNA construct of the invention can be used to produce products of interest. Therefore in another aspect, the invention is related to a method for producing a product of interest in the form of a dimeric fusion protein, which comprises growing a bacteria of the invention under conditions allowing the production and excretion to the culture medium of said product of interest in the form of a dimeric fusion protein. In a particular embodiment of the invention, said dimeric fusion protein comprises two products of interest. Therefore in a still more preferred aspect of the invention, a dimeric fusion protein would be obtained by means of expression of the nucleic acid sequences contained in at least one DNA construct of the invention, or at least one expression cassette of the invention, or at least one vector of the invention. The conditions for optimizing the culture of the bacteria of the invention will depend on the bacteria used.

If desired, the method for producing a product of interest provided by this invention further includes the isolation and purification of said dimeric fusion protein. In this case, the DNA construct of the invention further includes said previously defined seventh nucleic acid sequence comprising a nucleotide sequence coding for an amino acid sequence susceptible of being cleaved specifically by enzymatic or chemical means for the purpose of releasing the product of interest. In a particular embodiment, said nucleotide sequence codes for a protease recognition site, for example an enterokinase, endoprotease Arg-C, endoprotease Glu-C, endoprotease Lys-C, coagulation factor Xa, and the like. In another particular embodiment, said nucleic acid sequence codes for a site susceptible of being cleaved specifically by a chemical reagent such as, for example, cyanogen bromide, which cleaves methionine residue, or any other suitable chemical reagent.

In another aspect, the invention is related to a dimeric fusion protein that can be obtained by expression of at least one nucleic acid sequence contained in at least one DNA construct of the invention, wherein each monomer comprises:
(i) the amino acid sequence of a product of interest;
(ii) an amino acid sequence corresponding to a dimerization domain; and
(iii) the amino acid sequence of *E. coli* HlyA or of a fragment of said protein comprising the recognition signal of the *E*. *coli* hemolysin (Hly) transport system secretion mechanism.

More specifically, each monomer of the dimeric fusion protein of the invention comprises:
(i) a product of interest, for example an enzyme, an enzymatic inhibitor, a hormone, a molecule involved in cell adhesion and/or signaling and made up of domains, for example an immunoglobulin, an immunogenic antigen, such as a protein or an antigen fragment thereof from a pathogen, for example from a viral, bacterial or parasitic pathogen, etc., which may cause infections in human beings or animals, a therapeutic agent, for example a tumor-specific antigen, an auto-immune disease antigen, etc., or an immunoregulating molecule, for example a growth factor, a cytokine, such as an interleukin, an interferon, etc.; in a particular embodiment, said product of interest is a miniantibody susceptible of being used for therapeutic, diagnostic or research purposes;
(ii) a dimerization domain such as a peptide helix, a coiled coil structure, or generally any peptide sequence promoting dimerization in the proteins containing them. In a particular embodiment, said dimerization domain comprises the leucine zipper of the yeast transcription factor GCN4; and
(iii) the whole *E. coli* HlyA amino acid sequence, or alternatively an E. *coli* HlyA fragment comprising the recognition signal of the E. *coli* Hly transport system secretion mechanism.

Each monomer of the dimeric fusion protein of the invention may also contain, if so desired, (a) a spacer peptide between the product of interest and the dimerization domain; advantageously, said spacer peptide is a peptide with structural flexibility, for example a peptide containing repetitions of amino acid residues, such as Gly-Gly-Gly-Ser, or any other suitable amino acid residue repetition, or else the hinge region of an antibody; in a particular embodiment, said flexible spacer peptide comprises the hinge region of an antibody; and/or (b) a peptide to facilitate the isolation or purification of the peptide or fusion protein, for example a polyhistidine sequence, or a peptide sequence recognized by a monoclonal antibody and which can be useful for purifying the resulting fusion protein by immunoaffinity chromatography, for example tag peptides such as c-myc, HA, E, FLAG, and generally any other sequence recognized by an antibody; and/or (c) a peptide which allows the recognition of the peptide or fusion protein, for example a peptide sequence recognized by a monoclonal antibody and which can be useful for recognizing the resulting fusion protein by immunodetection techniques, for example tag peptides such as c-myc, HA, E, FLAG, and generally any other sequence recognized by an antibody; and/or (d) an amino acid sequence susceptible of being cleaved specifically by enzymatic means, for example an amino acid sequence forming a protease recognition site, for example an enterokinase, endoprotease Arg-C, endoprotease Glu-C, endoprotease Lys-C, coagulation factor Xa, and the like, or an amino acid sequence susceptible of being cleavedspecifically by a chemical reagent such as, for example, cyanogen bromide and the like.

The dimeric fusion protein production system provided by this invention is particularly useful for producing proteins involved in a binding activity, for example in protein-DNA or antigen-antibody interactions, since it may intensify their avidity.

In another still more preferred embodiment of the invention, the DNA constructs of the invention are useful for creating and expressing a library of dimeric proteins, for example of miniantibodies. A particular example of this embodiment would be to use the miniantibody dimers thus produced in processes for selecting molecules with a high capacity for binding to a given antigen.

In another embodiment of the invention, the dimeric protein production system is useful for producing heterodimers between two molecules with a binding capacity for different antigens or different epitopes of the same antigen, preferably two miniantibodies, or a miniantibody and another type of molecule, such as, though not limited to, a toxin, an anti-tumor drug, an enzyme or molecules involved in labeling or detection. Therefore, a particular example of this embodiment would be to use these dimers for tumor cell labeling or transporting molecules with anti-tumor activity to the tumor. The production of this type of heterodimers has significant applications in diagnosis and therapy.

An advantage of the system provided by this invention is based on the fact that it allows producing toxic proteins for a bacterial host. As is known, the expression by toxic protein recombinant methods for a bacterial host is very complicated or virtually impossible when said expressed toxic protein is not exported from the bacteria to the outside. With the method provided by this invention, a toxic or previously non-expressible protein, or one that is expressed at low levels, can be expressed in order to produce the desired protein in usable amounts.

The following example illustrates the invention without necessarily considering it to be limiting of the scope thereof.

### EXAMPLE 1

### Production of high affinity dimeric miniantibodies secreted by the E. coli hemolysin (Hly) transport system

This example describes the secretion of dimeric miniantibodies in supernatants of the *E. coli* culture which use the hemolysin (Hly) transport system. First, it was shown that dimerization can be achieved by genetic engineering in the Hly transport system. To that end, an amphipathic α helix (i.e. the leucine zipper domain of the yeast transcription factor GCN4) was inserted in the N-terminal end of a tagged version (E-tag) of the C-terminal domain of 23 kDa of hemolysin (EHlyA). It was verified that the resulting polypeptide (ZEHlyA) was effectively secreted by the E. *coli* cells and was accumulated in the culture medium as a stable dimer. Then the vectors derived from 'EHlyA and 'ZEHlyA were used for the secretion of the immunoglobulin V_{HH} domains obtained from camel antibodies. The V_{HH}-EHlyA and V_{HH-}ZEHlyA hybrids were secreted and found in the extracellular medium as monomers and dimers, respectively. When the dimeric V_{HH}-ZEHlyA dimeric molecules were compared with their monomeric homologues, they showed greater binding properties to their related antigen with a 10-fold increase in their functional affinity (avidity). This process allows easily obtaining high avidity monomeric and dimeric V_{HH} miniantibodies from supernatants of the E. *coli* culture, thus facilitating the high yield selection and purification of V_{HH} clones from large libraries.

### 1. MATERIALS AND METHODS

**Bacterial strains, growth and induction conditions.** The K-12 E. *coli* strains used were DH5αF' *(supE44 Δ(lacZYA-argf)*U169 *Φ*80*(lacZΔM15) hsdR17 recAl endAl gyrA96 thil relA1;* Invitrogen) for the cloning and propagation of the plasmids, and HB2151 (*Δlac-pro, ara, nal*^{*r*}*, thi,* F'*proAB lacl*^{*q*} *lacZΔM15*) [Carter, Pl, H. Bedouelle, and G. Winter 1985. Improved oligonucleotide site-directed mutagenesis using M13 vectors. Nucleic Acid Res. 13:4431-4443] for protein expression. The bacteria containing the plasmids indicated in each case were grown at 30°C on LB agar plates [Miller, J.H. 1992. A short course in bacterial genetics: a laboratory manual and handbook for *Escherichia coli* and related bacteria. Cold Spring Harbor Laboratory Press, Cold Spring Harbor, New York] containing 2% glucose (w/v) (to repress the *lac* promoter) and suitable antibiotics for selecting the plasmids. To induce the HlyA hybrids, individual colonies were inoculated in the liquid LB medium which contained 2% glucose (w/v) and were grown at 30°C or at 37°C until the optical density at 600 nm (OD₆₀₀ nm) reached a value of about 0.5. At this stage, the bacteria were collected by centrifugation, resuspended at the same density in LB that contained 0.3 mM isopropyl-1-thio-β-D-galactoside (IPTG) and incubated (at 30°C or at 37°C) with stirring (160 rpm) for a time period comprised between 4 and 16 h. The supernatants of the culture were collected after the elimination of the *E. coli* cells by centrifugation (10,000xg, 10 minutes) and 1/10 of the 10X concentrated phosphate buffered saline (PBS) was added [PBS: 8 mM Na₂HPO₄, 1.5 mM KH₂PO₄, 3 mM KCl, 137 mM NaCl, pH 7.0]. The supernatants of the culture were directly used to carry out immunoassays or they were stored at -80°C until their use. The antibiotics added to the culture medium for selecting the plasmids were ampicillin (Ap; 150 µg/ml) and chloramphenicol (Cm; 30 µg/ml).
**Plasmids and oligonucleotides.** Standard DNA handling and isolation methods, PCR amplification, and DNA sequencing were used [Ausubel, F.M., R. Brent, R.E. Kingston, D.D. Moore, J.G. Seidman, J.A. Smith, and K. Struhl 1994. Current Protocols in Molecular Biology. John Wiley & Sons, New York; Sambrook J., E Fritsch and T. Maniatis 1989. Molecular cloning, a Laboratory Manual. Cold Spring Harbor Laboratory Press, New York]. The oligonucleotides were obtained from Sigma Genosys (United Kingdom) or from Isogen bioscience BV (Netherlands). The plasmids pEHlyA (Ap^{r}), pEHlyA2-SD (Ap^{r}) and pVDL9.3 (Cm^{r}) have already been described [Fernández, L.A. and V. de Lorenzo. 2001. Formation of disulphide bonds during secretion of proteins through the periplasmic-independent type I pathway. Mol Microbiol. 40:332-46; Fernández, L.A., I. Sola, L. Enjuanes and V. de Lorenzo. 2000. Specific secretion of active single-chain Fv antibodies into the supernatants of *Escherichia coli* cultures by use of the hemolysin system. Appl Environ Microbiol. 66:5024-5029; Tzschaschel, B.D., C.A. Guzman, K.N. Timmis, and V. de Lorenzo 1996. An *Escherichia coli* hemolysin transport system-based vector for the export of polypeptides: Export of Shigalike toxin IleB subunit by *Salmonella typhimurium* aro4. Nature Biotechnology. 14:765-769]. The plasmid pZEHlyA (Ap^{r}) was obtained by inserting in the only pEHlyA *Sal*I site a 170 bp DNA fragment coding for the ZIP domain amplified by PCR and digested with *Sal*I*.* The map of the plasmid pZEHlyA is shown in Figure 6. PCR amplification of the ZIP domain was carried out with Vent DNA polymerase (New England Biolabs), using 1 ng of pCLZIP (Codon Genetic Systems, GmbH) as a template, and the oligonucleotides identified as SEQ ID NO: 5 and SEQ ID NO: 6, which incorporated two *SalI* sites flanking the amplified product, as primers. The plasmid pZEHlyA2-SD (Ap^{r}) was obtained by inserting in the only pEHlyA2-SD *Sal*I site the 170 bp DNA fragment coding for ZIP obtained by digestion with pZEHlyA *SalI.* The map of plasmid pZEHlyA2-SD is shown in Figure 7. The orientation of the ZIP DNA fragment which produced an internal insertion in the E-tagged C-HlyA domain of pZEHlyA and pZEHlyA2-SD after DNA sequencing was chosen. The DNA fragments of about 0.3 kb which encoded for the VHH, V_{amy} and Vₜₜₓ domains were amplified by means of PCR with Vent DNA polymerase, using 1 ng of the A100R3A2 (anti-α-amylase) or R3E5 (anti-tetanus vaccine) phagemids, respectively, as a template and the oligonucleotides identified as SEQ ID NO: 7 and SEQ ID NO: 8 as primers.

The amplified DNA products coding for V_{amy} and Vₜₜₓ contained the flanking restriction sites *Nco*I and SfiI, which allowed their cloning into the same pEHlyA2-SD and pZEHlyA2-SD sites, thus generating pV_{*amy*}HlyA, pV_{*ttx*}HlyA, pV_{*amy*}ZHlyA and pV_{*ttx*}ZHlyA. The maps of the plasmids pV_{*amy*}HlyA and pV_{*amy*}ZHlyA are shown in Figures 8 and 9, respectively. The phagemids A100R3A2 and R3E5 were provided by Dr. Henni Hoogenboon (Dyax Co., USA). Both phagemids are derivatives of pCANTAB6 (Cambridge Research Biochemicals) that contain the camelid V_{HH} domains cloned between the sites SfiI and *NotI.*
**Protein electrophoresis and immunoblotting.** Sodium dodecyl sulfate-polyacrylamide gel electrophoresis (SDS-PAGE) was carried out using 4% stacking gels and 10% separating gels (acrylamide:bisacrylamide 29:1; Bio-Rad), using the electrophoresis system Miniprotean® (Bio-Rad) and following standard protocols [Ausubel, F.M., R. Brent, R.E. Kingston, D.D. Moore, J.G. Seidman, J.A. Smith, and K. Struhl 1994. Current Protocols in Molecular Biology. John Wiley & Sons, New York; Fraile, S., F. Roncal, L.A. Fernández and V. de Lorenzo 2001. Monitoring Intracellular Levels of XylR in *Pseudomonas putida* with a Single-Chain Antibody Specific for Aromatic-Responsive Enhancer-Binding Proteins. J Bacteriol. 183:5571-9]. For the immunoblotting, the proteins were blotted on a polyvinylidene difluoride membrane (Immobilon-P, Millipore) using a semi-dry blotting electrophoresis equipment (Bio-Rad). The membrane was immobilized in MTP buffer (3% skim milk w/v, 0.1% Tween 20 v/v in PBS) and the E-tagged polypeptides with an anti-E monoclonal antibody labeled with peroxidase (0.2 µg/ml in MTP buffer; Amersham Bioscience) were detected. The bound antibody-POD conjugate was shown by means of chemiluminescence, as has already been described [Fraile, S., F. Roncal, L.A. Fernández and V. de Lorenzo 2001. Monitoring Intracellular Levels of XylR in *Pseudomonas putida* with a Single-Chain Antibody Specific for Aromatic-Responsive Enhancer-Binding Proteins. J Bacteriol. 183:5571-9]. The membrane was exposed on an X-ray film (X-OMAT®, Kodak) or on ChemicDoc® (Bio-Rad) for chemiluminescence quantification (Quantity-one® software; Bio-Rad). The concentrations of the secreted E-tagged HlyA polypeptides present in the supernatants of the E. *coli* culture were determined by the intensity of their corresponding protein bands in silver stained SDS-polyacrylamide gels [Ansorge, W. 1985. Fast and sensitive detection of protein and DNA bands by treatment with potassium permanganate. J. Biochem. Biophys. Methods. 11:13-20] and by means of immunoblotting using POD-labeled anti-E monoclonal antibody. Serial dilutions of purified E-tagged scFvs of unknown concentrations were used as standards in these experiments.
**Protein cross-linking.** Before their incubation with the bifunctional cross-linking agent disuccinimidyl glutarate (DSG, 7.7 Å spacer; Pierce), the E-tagged HlyA polypeptides present in the supernatants of the cultures were balanced in the same volume of PBS by ultrafiltering through a membrane with a 10 kDa cut-off (Microcon 10, Millipore) which eliminated the small compounds with free amino groups present in the culture medium. The cross-linking was carried out for 30 minutes at ambient temperature adding 40 or 130 µM DBS to 50 µl protein samples balanced in PBS. After this incubation, the cross-linking agent was inactivated with 50 mM Tris-HCl (pH 7.5) for 15 minutes and a volume of 2X concentrated SDS-PAGE sample buffer was added [Fraile, S., F. Roncal, L.A. Fernández and V. de Lorenzo 2001. Monitoring Intracellular Levels of XylR in *Pseudomonas putida* with a Single-Chain Antibody Specific for Aromatic-Responsive Enhancer-Binding Proteins. J Bacteriol. 183:5571-9]. After boiling for 5 minutes, 10 µl were loaded for the SDS-PAGE.
**Size exclusion chromatography.** The supernatants of the culture (about 0.2 ml) which contained 1X PBS were mixed with 2 mg of known mass standard protein (dissolved in 60 µl of H₂O) and were passed through a 1.5 m Bio-Gel A resin (Bio-Rad) packed in a column of 1 m in length and 1.5 cm in width. The gel filtration standards (Bio-Rad) were thyroglobulin (MW 670,000), bovine gamma globulin (MW 158,000), chicken ovalbumin (MW 44,000), equine myoglobin (MW 17,000) and vitamin B-12 (MW 1,350). The sample flow rate through the column was set at 0.2 ml/min using a peristaltic pump (P-1, Amersham Bioscience). The void volume of the column was calculated by means of eluting dextran blue 2000 (Amersham Bioscience). The elution of the protein standards through the column was monitored by means of UV light absorption (Uvicord S II, Amersham Bioscience). 1 ml fractions were collected (RediFrac collector, Amersham Bioscience) and were concentrated ten times by means of precipitation with 10% trichloroacetic acid (TCA) (w/v) and 10 µg of bovine serum albumin (BSA, Roche) which acted as a carrier. The presence of E-tagged HlyA proteins in these fractions was detected by means of Western blot using a POD-labeled anti-E monoclonal antibody (see above).
**Enzyme-Linked Immunosorbent Assay (ELISA).** The antigens (α-amylase or ovalbumin; Sigma) were absorbed for 1 hour at 37°C on 96-well microtiter immunoplates (Maxisorb, Nunc) at 200 µg/ml in PBS. The antigen excess was washed and the plates were immobilized for 16 hours at 4°C in MTP buffer (see above). The miniantibodies were diluted in MTP buffer, added to the wells at the concentrations indicated in each case and incubated for 1 hour at ambient temperature. Then, the non-bound antibodies were eliminated by means of four washings of the wells with PBS that contained 0.1% Tween 20 (v/v). The POD-labeled anti-E monoclonal antibody conjugate (0.2 µg/ml in MTP buffer) was added to the wells and further incubated for 1 hour at ambient temperature in order to detect the E-tagged bound miniantibodies. After washing as before, the plates were developed using o-phenylenediamine (Sigma). The reaction was left to continue for 10 minutes in the dark, it was stopped with 0.6 N HCl and the OD at 490 nm of the wells was determined (Benchmark microplate reader, Bio-Rad).

### 2. RESULTS

### Dimerization by genetic engineering of the HlyA secretion signal.

It was first studied if C-HlyA dimerization could be carried out by genetic engineering without affecting its secretion. A short domain of about 6 kDa called ZIP was chosen, which had been used for scFv dimerization in the E. *coli* periplasm [Kerschbaumer, R.J., S. Hirschl, A. Kaufmann, M. Ibl, R. Koenig and G. Himmler. 1997. Single-chain Fv fusion proteins suitable as coating and detecting reagents in a double antibody sandwich enzyme-linked immunosorbent assay. Anal Biochem. 249:219-27; Pack, P. and Plückthun. 1992. Miniantibodies: use of amphipathic helices to produce functional, flexibly linked dimeric FV fragments with high avidity in *Escherichia coli.* Biochemistry. 31:1579-84] were chosen for the insertion thereof in C-HlyA. The ZIP domain consists of an amphipathic helix forming the leucine zipper of the yeast transcription factor GCN4, flanked at its N-terminal end by a peptide hinge area derived from mouse IgG3, and at its C-terminal end by a polyhistidine tag (6xhis). A DNA fragment coding for the ZIP domain was inserted internally close to the N-terminal end of the E-tagged C-HlyA (EHlyA) version of about 27 kDa present in the plasmid pEHlyA (Figure 1A). The resulting plasmid, pZEHlyA, codes for a polypeptide of about 33 kDa (called ZEHlyA) containing the E-tagged ZIP and C-HlyA domains (Figures 1A and 1B).

The production of ZEHlyA and EHlyA as a control without ZIP was induced in wild-type TolC⁺ cell cultures of *E. coli* (for example, the HB2151 strain) which incorporated pVDL9.3, which codes for HlyB and HlyD, and which housed pZEHlyA or pEHlyA, respectively. As shown in Figure 1C, both polypeptides were found in similar levels (about 10 µg/ml) in the supernatants of the E. *coli* cultures grown at 37°C after the 4 hour induction with 0.3 mM IPTG. These proteins were detected by virtue of the E-tagged polypeptide incorporated in the sequences with POD-labeled anti-E monoclonal antibody (Methods). The secretion of both HlyA-derived polypeptides was specific and dependent on the expression of the HlyB and HlyD components by E. *coli* (data not shown) [Fernández, L.A., and V. de Lorenzo 2001. Formation of disulphide bonds during secretion of proteins through the periplasmic-independent type I pathway. Mol Microbiol. 40:332-46]. This result indicated that the presence of the ZIP dimerization domain had no effect on the efficacy of the C-HlyA signal exportation.

Then, the oligomerization state of the secreted polypeptides was studied. Aliquot samples which contained the secreted polypeptides EHlyA or ZEHlyA were incubated with the bifunctional cross-linking agent disuccinimidyl glutarate (DSG) and then they were subjected to denaturing SDS-PAGE and to immunoblotting with POD-labeled anti-E monoclonal antibody (Methods). In this experiment only the samples of ZEHlyA at low DSG concentration (40 µM) were cross-linked in order to form a protein band with an apparent molecular mass (Mr) of about 66 kDa (Figure 2, lane 5), which was pursuant to the expected size for a ZEHlyA dimer. The highest DSG concentration (130 µM) intensified the intensity of the band corresponding to dimeric ZEHlyA (Figure 2, lane 6), whereas it only had less reactivity over the control EHlyA (Figure 2, lane 3).

The ZEHlyA dimerization was also demonstrated by means of size exclusion chromatography. Aliquot samples of supernatants of the culture containing secreted EHlyA or ZEHlyA were separated in a gel filtration column with an exclusion limit of 1,500 kDa, together with known mass proteins used as standards (Methods). As shown in Figure 3A, ZEHlyA showed an apparent Mr of about 66 kDa in the gel filtration chromatography, whereas EHlyA had an apparent Mr of about 32 kDa in the same conditions. It is important that a single peak was detected for each protein (Figure 3B), which indicates that both polypeptides were present as stable monomers (EHlyA) and dimers (ZEHlyA) in solution. Taken together, these results demonstrated that protein dimerization could be obtained by means of the incorporation of amphipathic helixes in C-HlyA without interfering with their secretion by the Hly transporter.
**Secretion of dimeric miniantibodies by the Hly system of *E. coli.*** In view of the previously obtained results, it was then studied whether the dimeric antibody fragments could be secreted by means of the Hly system. First a plasmid called pZEHlyA2-SD was constructed in order to generate internal fusions between the recombinant antibodies fragments lacking the N-SP domain and ZEHlyA. This plasmid is a derivative of pEHlyA2-SD [Fernández, L.A., I. Sola, L. Enjuanes and V. de Lorenzo. 2000. Specific secretion of active single-chain Fv antibodies into the supernatants of *Escherichia coli* cultures by use of the hemolysin system. Appl Environ Microbiol. 66:5024-5029] in which a DNA fragment coding for the ZIP domain was inserted at a single SalI site between the polybinding sequence and the E-tagged C-HlyA domain (Method). Two camel V_{HH} antibodies were chosen, against α-amylase *(amy)* or the anti-tetanus vaccine (ttx), in order to determine their expression as hybrids with the 'EHlyA and 'ZEHlyA remains (Figures 4A and 4B) . The use of camel V_{HH} antibodies as fusion pairs was due to their small size (about 15 kDa) and their low tendency to form protein aggregates [Muyldermans, S. 2001. Single domain camel antibodies: current status. J Biotech. 74:277-302; Plückthun, A. and P. Pack. 1997. New protein engineering approaches to multivalent and bispecific antibody fragments. Immunotechnology. 3:83-105], which could interfere with the analysis of the dimerization obtained by the ZIP domain (see Discussion). The *E. coli* cells HB2151 (pVDL9.3) were transformed with a plasmid coding for the V_{HH}-HlyA hybrid (pV_{*amy*}HlyA, pV_{*amy*}ZHlyA, pV_{*ttx*}HlyA or pV_{*ttx*}ZHlyA) and were induced for 4 hours by the addition of 0.3 mM IPTG to the liquid cultures grown in LB at 30 or 37°C. The secreted polypeptides V_{amy}HlyA and V_{amy}ZHlyA were subsequently detected in the supernatants of the corresponding E. *coli* cultures by Western blot with POD-labeled anti-E monoclonal antibody (Figure 4C). In these conditions, the final concentration of V_{*amy*}HlyA and V_{*amy*}ZHlyA was about 2 µg/ml at 37°C, and was reduced by about two times in the cultures which grew at 30°C. Similar results were obtained with V_{*ttx*}HlyA and V_{*ttx*}ZHlyA (data not shown).

The oligomerization state of the secreted V_{HH}-HlyA hybrids was subjected to assay by means of gel filtration chromatography (Figure 4D). Aliquot samples of supernatants of E. *coli* cultures which contained V_{amy}HlyA or V_{amy}ZHlyA were loaded in a gel filtration column (1,500 kDa exclusion limit) together with known mass proteins. It can be deduced from their elution profiles (Figure 4D) that the V_{*amy*}HlyA hybrid had an apparent Mr of about 40 kDa, which was completely pursuant to the expected mass for a monomer of this polypeptide. In contrast, V_{amy}ZHlyA showed an apparent Mr of about 95 kDa, which is about twice the mass expected for its monomer (i.e. 47 kDa). It must be mentioned that the temperature at which the *E. coli* cultures were induced (30°C or 37°C) had no effect on the chromatographic behavior of these samples. Therefore, the secretion of the monomeric or dimeric camel V_{HH} antibodies can be produced by fusing them to the EHlyA or ZEHlyA remains, respectively.

Then it was tested whether the dimerization improved the functional binding properties of V_{*amy*}ZHlyA. For this purpose, the binding of monomeric V_{*amy*}HlyA and of dimeric V_{*amy*}ZHlyA to α-amylase was compared by means of ELISA. In these experiments, serial dilutions of supernatants of E. *coli* cultures which contained identical amounts of V_{*amy*}HlyA or V_{*amy*}ZHlyA were incubated with ELISA plates coated with α-amylase or ovalbumin (as control antigen). After the washing, the bound miniantibodies were detected with the POD-labeled anti-E monoclonal antibody conjugate and the reading was carried out at OD₄₉₀ₙₘ (Methods). The specific binding of α-amylase was demonstrated by incubating these plates with V_{*ttx*}HlyA and V_{*ttx*}ZHlyA. Figure 5 shows the result of a prototype ELISA of these experiments. The prior binding to ovalbumin (in all cases OD₄₉₀ₙₘ ≤ 0.05) was subtracted from the submitted values. As indicated, the dimeric V_{*amy*}ZHlyA molecule had a greater functional affinity for α-amylase than monomeric V_{*amy*}HlyA. No α-amylase binding was observed with the V_{*ttx*} control derivatives (Figure 5), nor with the polypeptides EHlyA and ZEHlyA (data not shown). Generally, at least a ten times greater concentration of V_{amy}HlyA was required to achieve α-amylase biding signals similar to those obtained with V_{amy}ZHlyA. Furthermore, in the saturation concentration of both antibodies (about 0.5 µg/ml), the binding obtained with V_{*amy*}ZHlyA reached a higher plateau level. Therefore, dimerization of V_{*may*}ZHlyA induces an avidity effect on this miniantibody which is reflected in a higher functional binding affinity for its antigen.

### 3. DISCUSSION

Dimerization is a property which is often desired to be obtained by genetic engineering in proteins when a binding activity is involved (for example, in protein-DNA or antigen-antibody interactions) given that it may intensify their functional affinity (avidity).

This example shows obtainment for the first time, by means of genetic engineering, of the dimerization of the proteins secreted by the E. *coli* hemolysin transport system and this technology has been used to produce high avidity miniantibodies derived from camel V_{HH} antibodies.

The obtained results demonstrate that the incorporation of an autodimerization amphipathic helix at the N-terminal end of C-HlyA does not interfere with the secretion of Hly and allows the dimerization of the secreted polypeptide. As shown, the dimerization may intensify the avidity of the binding of the secreted polypeptide derivative of C-HlyA. Furthermore, it may also have other applications, such as the molecular association of several antigens and/or adjuvants produced by live bacterial strains, or the combination of different biological activities for the generation of bi-specific molecules (for example, antigen binding and complement recruiting).

High avidity dimeric scFvs have been produced in the periplasm of E. *coli* cells by inserting amphipathic helixes at their C-terminal end. Due to the tendency of some scFvs to form high molecular weight protein aggregates and dimers, smaller antibody fragments were used.

Camel V_{HH} antibodies have received a great deal of attention due to their better solubility and their simpler structure, which facilitates their amplification and cloning. It is worth pointing out that the changes carried out do not decrease the affinity or the specificity of camel V_{HH} antibodies due to the presence of extremely variable complementarity determining regions (CDR) offsetting the loss of diversity caused by the absence of a domain. Camel antibodies have also demonstrated an extraordinary potential as enzymatic inhibitors given that their large CDRs can reach hidden active sites in enzymes. Furthermore, the similarity between camel V_{HH} antibodies and sequences of the human VH3 family is allowing the generation of libraries of phages of the camelized human V_{H} domains and of the humanized camel V_{HH} antibodies

The benefits set forth above motivated the inventors to use the VHH domains for their secretion by the E. *coli* hemolysin translocator. The obtained results show that the functional camel antibodies both in the monomeric and dimeric form, can be recovered from the supernatants of the *E. coli* culture at levels similar to those obtained in their periplasmic expression (about 1 mg/liter of culture at OD₆₀₀ₙₘ =1). Furthermore, the dimerization caused by ZEHlyA induced a ten-fold increase in the functional affinity of V_{*amy*}*.* This value is within the expected interval produced by the change of monovalent antibodies to divalent antibodies. In conclusion, this data demonstrates that the Hly secretion system can be used for the secretion of high avidity dimeric miniantibodies and polypeptides. The simplicity of this technology can be extremely useful for the high-yield selection of antibody libraries.

## Claims

**1.** A DNA construct comprising:
a) a first nucleic acid sequence containing the nucleotide sequence coding for a product of interest;
b) a second nucleic acid sequence containing the nucleotide sequence coding for a dimerization domain; and
c) a third nucleic acid sequence containing the nucleotide sequence coding for E. *coli* α-hemolysin (HlyA) or for a fragment of said protein comprising the recognition signal of the *E. coli* Hly transport system secretion mechanism, or a nucleotide sequence coding for a homologous gene, or a nucleotide sequence coding for a natural or artificial variant of HlyA or of a fragment thereof comprising the recognition signal of the *E*. *coli* Hly transport system secretion mechanism.

**2.** DNA construct according to claim 1, wherein the 3' end of said first nucleic acid sequence is bound to the 5' end of said second nucleic acid sequence, and the 3' end of said second nucleic acid sequence is bound to the 5' end of said third nucleic acid sequence.

**3.** DNA construct according to claim 1, wherein said product of interest is chosen from enzymes, enzymatic inhibitors, hormones, molecules involved in cell adhesion and/or signaling, molecules involved in detection or labeling, molecules made up of domains, immunogenic antigens, therapeutic agents, and immunoregulating molecules.

**4.** DNA construct according to claim 3, wherein said product of interest is chosen from tumor-specific antigens, auto-immune disease antigens, growth factors, cytokines, interleukins, interferons, and miniantibodies.

**5.** DNA construct according to claim 1, wherein said dimerization domain comprises a peptide helix or a coiled coil structure.

**6.** DNA construct according to claim 5, wherein said dimerization domain comprises a leucine zipper.

**7.** DNA construct according to claim 5, wherein said dimerization domain comprises the leucine zipper of the yeast transcription factor GCN4.

**8.** DNA construct according to claim 1, wherein said third nucleic acid sequence is chosen from:
a) the nucleotide sequence coding for HlyA of *E. coli;*
b) a nucleic acid sequence comprising the nucleotide sequence coding for the last 60 amino acids of the C-terminal end of HlyA of *E. coli;*
c) a nucleic acid sequence made up of a nucleotide sequence coding for the last 60 amino acids of the C-terminal end of HlyA of *E. coli;*
d) the nucleotide sequence identified as SEQ ID NO: 1; and
e) a nucleotide sequence coding for the amino acid sequence identified as SEQ ID NO: 2.

**9.** DNA construct according to claims 1 and 2, further comprising a fourth nucleic acid sequence coding for a spacer peptide located between said first and second nucleic acid sequences, wherein the 5' end of said fourth nucleic acid sequence is bound to the 3' end of said first nucleic acid sequence, and the 3' end of said fourth nucleic acid sequence is bound to the 5' end of said second nucleic acid sequence.

**10.** DNA construct according to claim 9, wherein said spacer peptide comprises amino acid residue repetitions, preferably Gly-Gly-Gly-Ser repetitions.

**11.** DNA construct according to claim 9, wherein said spacer polypeptide is a hinge region of an antibody.

**12.** DNA construct according to claim 1, further comprising a fifth nucleic acid sequence coding for a polypeptide susceptible of being used for isolation or purification purposes.

**13.** DNA construct according to claim 12, wherein said polypeptide susceptible of being used for isolation or purification purposes comprises a polyhistidine sequence or a polypeptide sequence recognized by a monoclonal antibody and which can be useful for purifying the resulting fusion protein by immunoaffinity chromatography.

**14.** DNA construct according to claim 12, wherein said fifth nucleic acid sequence is located between said second and third nucleic acid sequence ordered according to claim 2, wherein the 5' end of said fifth nucleic acid sequence is bound to the 3' end of said second nucleic acid sequence, and the 3' end of said fifth nucleic acid sequence is bound to the 5' end of said third nucleic acid sequence.

**15.** DNA construct according to claim 1, further comprising a sixth nucleic acid sequence coding for a peptide susceptible of being used for recognition purposes.

**16.** DNA construct according to claim 15, wherein said peptide susceptible of being used for recognition purposes comprises a peptide sequence recognized by a monoclonal antibody and can be useful for recognizing the resulting fusion protein by immunodetection techniques.

**17.** DNA construct according to claim 15, wherein said peptide susceptible of being used for recognition purposes comprises the E epitope sequence.

**18.** DNA construct according to claim 15, wherein said sixth nucleic acid sequence is located between said second and third nucleic acid sequences ordered according to claim 2, wherein the 5' end of said sixth nucleic acid sequence is bound to the 3' end of said second nucleic acid sequence, and the 3' end of said sixth nucleic acid sequence is bound to the 5' end of said third nucleic acid sequence.

**19.** DNA construct according to claims 12 or 15, wherein said fifth and sixth nucleic acid sequences are separated from one another.

**20.** DNA construct according to claims 12 or 15, wherein said fifth and sixth nucleic acid sequences are bound to one another.

**21.** DNA construct according to claim 20, wherein said fifth and sixth nucleic acid sequences are located between the second and third sequences ordered according to claim 2.

**22.** DNA construct according to claim 1, further comprising a seventh nucleic acid sequence comprising a nucleic acid sequence coding for an amino acid sequence susceptible of being cleaved specifically by enzymatic or chemical means.

**23.** DNA construct according to claim 22, wherein said seventh nucleic acid sequence comprises a nucleotide sequence coding for a protease recognition site.

**24.** DNA construct according to claim 23, wherein said protease is chosen from an enterokinase, endoprotease Arg-C, endoprotease Glu-C, endoprotease Lys-C and coagulation factor Xa.

**25.** DNA construct according to claim 22, wherein said seventh nucleic acid sequence comprises a nucleotide sequence coding for a site susceptible of being cleaved specifically by a chemical reagent.

**26.** DNA construct according to claim 25, wherein said chemical reagent is cyanogen bromide.

**27.** DNA construct according to claim 22, wherein said seventh nucleic acid sequence is located in any position between the second and third nucleic acid sequences ordered according to claim 2.

**28.** An expression cassette comprising a DNA construct according any of the previously claims operatively bound to an expression control sequence.

**29.** Expression cassette according to claim 28, wherein said expression control sequence comprises a promoter sequence, a transcriptional regulator coding sequence, a ribosome binding sequence (RBS) and/or a transcription terminating sequence.

**30.** Expression cassette according to claims 28 and 29, wherein said expression control sequence is functional in bacteria.

**31.** A vector comprising at least one DNA construct according to any of claims 1 to 27 or at least one expression cassette according to any of claims 28 to 30.

**32.** Vector according to claim 31, further comprising a marker.

**33.** Vector according to claim 32, wherein said marker comprises an antibiotic resistance gene or a toxic compound resistance gene.

**34.** A gram-negative bacteria comprising a DNA construct according to any of claims 1 to 27, or an expression cassette according to any of claims 28 to 30.

**35.** A gram-negative bacteria comprising at least one DNA construct according to any of claims 1 to 27, or at least one expression cassette according to any of claims 28 to 30, or at least one vector according to any of claims 31 to 33.

**36.** Bacteria according to claim 35, chosen from *Escherichia coli, Salmonella typhimurium, Pseudomonas aeruginosa* and *Pseudomonas putida.*

**37.** A dimeric fusion protein obtainable by expression of the nucleic acid sequences contained in a DNA construct according to any of claims 1 to 27.

**38.** A dimeric fusion protein obtainable by expression of the nucleic acid sequences contained in a DNA construct according to any of claims 1 to 27, or at least one expression cassette according to any of claims 28 to 30, or at least one vector according to claims 31 to 33.

**39.** Fusion protein according to claims 37 and 38, wherein each monomer comprises:
(i) the amino acid sequence of a product of interest.
(ii) an amino acid sequence corresponding to a dimerization domain; and
(iii) the amino acid sequence of α-hemolysin (HlyA) of *Escherichia coli* or of a fragment of said protein comprising the recognition signal of the *E. coli* hemolysin (Hly) transport system secretion mechanism.

**41.** Fusion protein according to claim 40, wherein each monomer comprises:
(i) a product of interest chosen from an enzyme, an enzymatic inhibitor, a hormone, a molecule involved in cell adhesion and/or signaling, molecules involved in detection or labeling, molecules made up of domains, an immunogenic antigen, a therapeutic agent, an immunoregulating molecule;
(ii) a dimerization domain chosen from a peptide helix and a coiled coil structure; and
(iii) the whole E. *coli* HlyA amino acid sequence, or an E. *coli* HlyA fragment comprising the recognition signal of the *E. coli* Hly transport system secretion mechanism.

**41.** Fusion protein according to claim 41, wherein each monomer comprises:
(i) a product of interest chosen from a tumor-specific antigen, an auto-immune disease antigen, a growth factor, a cytokine, an interleukin, an interferon and a miniantibody;
(ii) a dimerization domain chosen from a peptide helix and a coiled coil structure; and
(iii) the whole *E. coli* HlyA amino acid sequence, or an *E. coli* HlyA fragment comprising the recognition signal of the E. *coli* Hly transport system secretion mechanism.

**42.** Fusion protein according to claims 37 to 41, wherein each monomer further comprises (a) a spacer peptide between the product of interest and the dimerization domain; and/or (b) a peptide to facilitate the isolation and purification of the peptide or fusion protein; and/or (c) a peptide which allows recognition of the peptide or fusion protein; and/or (d) an amino acid sequence susceptible of being cleaved specifically by enzymatic or chemical means.

**43.** Fusion protein according to claim 42, wherein each monomer comprises (a) a peptide containing Gly-Gly-Gly-Ser repetitions or the hinge region of an antibody; and/or (b) a polyhistidine sequence or a peptide sequence recognized by a monoclonal antibody and which may be useful for purifying the resulting fusion protein by immunoaffinity chromatography; and/or (c) a peptide sequence recognized by a monoclonal antibody and which may be useful for recognizing the resulting fusion protein by immunodetection techniques; and/or (d) an amino acid sequence forming a recognition site of an enterokinase, endoprotease Arg-C, endoprotease Glu-C, endoprotease Lys-C or coagulation factor Xa, or an amino acid sequence susceptible of being cleaved specifically by a chemical reagent.

**44.** A method for producing a product of interest in the form of a dimeric fusion protein according to any of claims 37 to 43, comprising growing a bacteria according to claims 34 to 36 under conditions allowing the production and excretion of said product of interest to the culture medium in the form of a dimeric fusion protein.

**45.** Method according to claim 44 for producing a dimeric fusion protein, comprising two products of interest.

**46.** Method according to claims 44 and 45, further comprising the isolation and purification of said dimeric fusion protein.

**47.** Use of a DNA construct according to claims 1 to 27 for the creation of a dimeric protein library.

**48.** Use of the library of the previous claim for choosing molecules with the capacity to bind to a given antigen.

**49.** Fusion protein according to claims 37 to 43, for the use thereof in therapy.

**50.** Fusion protein according to claims 37 to 43 for the use thereof in *in vitro* or in vivo diagnosis.
